Europäisches Patentamt

European Patent Office

Office européen des brevets

(19)

(11) **EP 0 658 204 B1**

(12) **EUROPEAN PATENT SPECIFICATION**

(45) Date of publication and mention
of the grant of the patent:
**05.06.2002 Bulletin 2002/23**

(51) Int Cl.[7]: **C12N 15/54**, A61K 38/45
// C12N9/12

(21) Application number: **93921241.1**

(22) Date of filing: **27.08.1993**

(86) International application number:
**PCT/US93/08131**

(87) International publication number:
**WO 94/05794 (17.03.1994 Gazette 1994/07)**

(54) **DNA ENCODING THE HEME-REGULATED EUKARYOTIC INITIATION FACTOR 2$g(a) KINASE**

DNA KODIEREND FÜR DIE HÄM-REGULIERTE KINASE DES EUKARYONTISCHEN
INITIATIONSFAKTORS 2-ALPHA

ADN CODANT LA 2$g(a) KINASE DU FACTEUR D'INITIATION EUCARYOTIQUE A REGULATION
HEMIQUE

(84) Designated Contracting States:
**AT BE CH DE DK ES FR GB GR IE IT LI LU MC NL
PT SE**

(30) Priority: **31.08.1992 US 938782**

(43) Date of publication of application:
**21.06.1995 Bulletin 1995/25**

(73) Proprietor: **MASSACHUSETTS INSTITUTE OF
TECHNOLOGY
Cambridge, MA 02139 (US)**

(72) Inventors:
• **CHEN, Jane-Jane
Belmont, MA 02178 (US)**
• **LONDON, Irving M.
Cambridge, MA 02138 (US)**

(74) Representative: **Bassett, Richard Simon et al
Eric Potter Clarkson,
Park View House,
58 The Ropewalk
Nottingham NG1 5DD (GB)**

(56) References cited:
• **PROCEEDINGS OF THE NATIONAL ACADEMY
OF SCIENCES OF USA vol. 88, no. 17, 1
September 1991, WASHINGTON US pages 7729
- 7733 CHEN, J.-J. ET AL. 'Cloning of the cDNA
of the heme-regulated eukaryotic initiation
factor 2 alpha (eIF-2 alpha) kinase of rabbit
reticulocytes: homology to yeast GCN2 protein
kinase and human
double-stranded-RNA-dependent eIF-2 alpha
kinase'**
• **JOURNAL OF CELL BIOLOGY vol. 115, no. 3/2,
November 1991, NEW YORK, USA page 435A
YANG, J. ET AL. 'Structure-function study of
heme-regulated eukaryotic initiation factor 2
alpha kinase by site-directed mutagenesis'**
• **PROCEEDINGS OF THE NATIONAL ACADEMY
OF SCIENCES OF USA vol. 88, January 1991,
WASHINGTON US pages 315 - 319 CHEN, J.-J.
ET AL. 'Amino acid microsequencing of internal
tryptic peptides of heme-regulated eukaryotic
initiation factor 2 alpha subunit kinase:
homology to protein kinases' cited in the
application**
• **JOURNAL OF BIOLOGICAL CHEMISTRY vol.
267, no. 16, 5 June 1992, BALTIMORE, MD US
pages 11500 - 11507 M NDEZ, R. ET AL.
'Regulation of heme-controlled eukaryotic
polypeptide chain initiation factor 2
alpha-subunit kinase of reticulocyte lysates'**

**Description**

**Background of the Invention**

The present invention relates to heme-regulated eukaryotic initiation factor 2α kinase, certain modifications thereof and methods of use thereof in inhibition of cellular proliferation.

Heme controls the synthesis of protein in reticulocytes. In heme-deficiency, there is diminished initiation of protein synthesis with disaggregation of polyribosomes. The principal mechanism of the inhibition of initiation of protein synthesis is the phosphorylation of the α-subunit of the eukaryotic initiation factor 2, eIF-2α. In addition to heme-deficiency, oxidized glutathione (GSSG) and low levels of double stranded RNA inhibit initiation by promoting phosphorylation of eIF-2α.

The translation of mRNA in eukaryotic cells occurs in the cytoplasm. In the first step of initiation, free 80 S ribosomes are in equilibrium with their 40 S and 60 S subunits. In the presence of eIF-3, 40 S subunits bind the eIF-3 and eIF-4C to form a 43 S ribosomal complex; the binding of eIF-3 and eIF-4C to the 40 S subunit inhibits the joining of the 60 S subunit.

In the next step, eIF-2 binds GTP and the initiator tRNA, Met-tRNA$_f$, in a ternary complex. The binding by eIF-2 is specific for both guanine nucleotides and for Met-tRNA$_f$. The ternary complex now binds to the 43 S ribosomal complex to form the 43 S preinitiation complex. The 43 S preinitiation complex binds mRNA in an ATP-dependent reaction in which eIF-4A, eIF-4B, and eIF-4F form a complex with the mRNA. The product of the binding of mRNA to the 43 S structure is bound close to the ribosome and the AUG initiator codon is downstream from the cap structure.

The joining of the 48 S preinitiation complex and the 60 S subunit is catalyzed by eIF-5 which has a ribosome-dependent GTPase activity. The joining reaction is accompanied by the release of the initiation factors eIF-3 and eIF-4C, eIF-2 is translocated to 60 S subunit as a binary complex, eIF2-GDP. The product of the joining reaction is the 80 S initiation complex. Formation of the active 80 S initiation complex is the final step in initiation. The Met-tRNA$_f$ is positioned in the P (peptidyl) site on the ribosome for the start of polypeptide elongation.

The sequence of steps in the process of initiation affords several opportunities for regulation. These include the recycling of eIF-2 after its release as the eIF-2-GDP complex; the formation of the ternary complex; and the relative affinities of mRNAs for eIF-2 and for eIF-4A, -4B, and -4F in determining the relative rates of translation of the mRNAs.

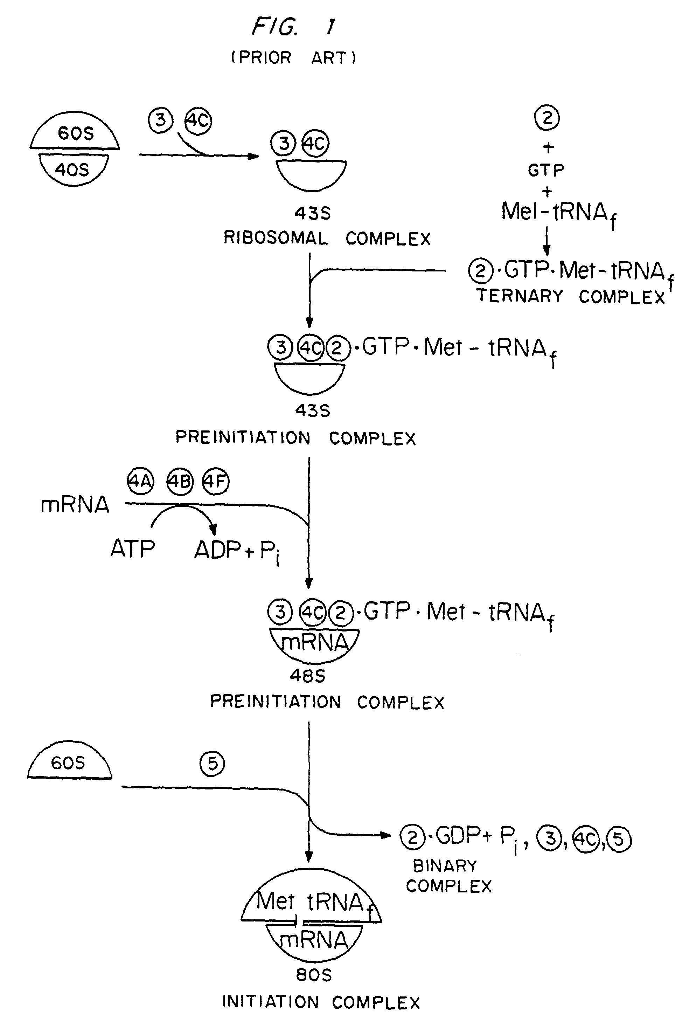

A schematic summary of eukaryotic initiation is shown in Figure 1. Heme-deficiency inhibited initiation of protein synthesis is characterized by a brief period of control linear synthesis, followed by an abrupt decline in this rate and by disaggregation of polyribosomes, associated with a decrease in the formation of the eIF-2-Met-tRNA$_f$-GTP ternary complex and the 40 S-eIF-2Met-tRNA$_f$-GTP 43 S initiation complex. The fundamental mechanism for the inhibition is the activation of cAMP independent protein kinases that specifically phosphorylate the 38-kDa α-subunit of eIF-2 (eIF-2α). Dephosphorylation of eIF-2α accompanies the recovery of protein synthesis upon addition of hemin to inhibited heme-deficient lysates.

The heme-regulated eukaryotic initiation factor 2α (eIF-2α) kinase, also called heme-regulated inhibitor (HRI), plays a major role in this process. HRI is a cAMP-independent protein kinase that specifically phosphorylates the α subunit (eIF-2α) of the eukaryotic initiation factor 2 (eIF-2). Phosphorylation of eIF-2α in reticulocyte lysates results in the binding and sequestration of reversing factor RF, also designated as guanine nucleotide exchange factor or eIF-2B, in a RF-eIF-2(αP) complex; the unavailability of RF, which is required for the exchange of GTP for GDP in the recycling of eIF-2 and in the formation of the eIF-2-Met-tRNA$_f$-GTP ternary complex, resulting in the cessation of the initiation of protein synthesis.

Although the mechanism of regulation of protein synthesis by HRI has been extensively studied, little is known about the structure and regulation of HRI itself. Chen, J.-J., et al., *Proc. Natl. Acad. Sci.,* USA 88:315-319 (1991) previously reported the amino acid sequences of three tryptic peptides of heme-reversible HRI. HRI peptide P-52 contains the sequence Asp-Phe-Gly, which is the most highly conserved short stretch in conserved domain VII of protein kinases as presented by Hanks, Quinn, and Hunter, *Science* 241:42-52 (1988). The N-terminal 14 amino acids of HRI peptide P-74 show 50-60% identity to the conserved domain IX of kinase-related transforming proteins. These findings are consistent with the autokinase and eIF-2α kinase activities of HRI. As reported by Pal et al., *Biochem.* 30: 2555-2562 (1991), this protein appears to be erythroid-specific and antigenically different in different species.

In view of the activity and relationships of HRI to other protein kinases involved in cellular transformation, it would be advantageous to have the nucleic acid sequence encoding HRI. However, since the gene is only expressed during a very limited time period, i.e., during erythroid differentiation, and in an extremely minuscule amount, this was not a simple process. Moreover, even though three peptides isolated by tryptic digest had been sequenced, it was not clear if these were from HRI or from a contaminant of the HRI preparation. Obtaining a library containing a full length HRI cDNA is also difficult.

# EP 0 658 204 B1

## Summary of the Invention

A first aspect of the invention provides a pharmaceutical composition comprising heme-regulated eukaryotic initiation factor 2α kinase or the kinase modified in the heme binding region in the 170 N-terminal amino acids or the kinase insertion sequence to decrease regulation by heme in combination with a suitable pharmaceutical carrier for administration to a patient wherein the kinase or modified kinase is in a dosage effective to induce cellular differentiation, inhibit cellular proliferation or inhibit infection.

A second aspect of the invention provides use of a heme-regulated eukaryotic initiation factor 2α kinase or the kinase modified in the heme binding region in the 170 N-terminal amino acids or the kinase insertion sequence to decrease regulation by heme in the manufacture of a medicament for inducing cellular differentiation or for inhibiting cellular proliferation in a patient.

A third aspect of the invention provides use of heme-regulated eukaryotic initiation factor 2α kinase or the kinase modified in the heme binding region in the 170 N-terminal amino acids or the kinase insertion sequence to decrease regulation by heme in the manufacture of a medicament for inhibiting infection in a patient.

A fourth aspect of the invention provides use of a heme-regulated eukaryotic initiation factor 2α kinase or the kinase modified in the heme binding region in the 170 N-terminal amino acids or the kinase insertion sequence to decrease regulation by heme in the manufacture of a medicament for regulating cell proliferation in a patient.

The cDNA which encodes heme-regulated eIF-2α kinase (HRI) has been cloned from a lambda Zap II cDNA library of rabbit reticulocytes. The rabbit HRI cDNA is highly homologous to human HRI and hybridizes to the human HRI DNA under moderately stringent conditions. The rabbit HRI cDNA contains 2729 nucleotides and encodes 626 amino acids. *In vitro* translation of HRI mRNA transcribed from HRI cDNA yields a 90 kDa polypeptide with eIF-2α kinase activity. This 90 kDa polypeptide is recognized by an anti-HRI non-species specific monoclonal antibody. These properties are characteristic of authentic HRI.

Since HRI is a potent inhibitor of protein synthesis, it is anti-proliferative in nature. In addition, the unusually high degree of homology of HRI to three protein kinases involved in the regulation of cell division suggests that HRI may play a direct role in the regulation of cell division. The availability of HRI cDNA provides a means to study the regulation and the structure and function relationship of HRI. Furthermore, since regulation of protein synthesis is vital for cell growth and differentiation, the cDNA can be inserted into cells to manipulate proliferation and differentiation, especially of cells that are proliferating in an uncontrolled manner or characterized by arrested differentiation, such as some of the types of cancers.

Initiation of protein synthesis can also be regulated by another eIF-2α kinase which is activated by double-stranded RNA (dsI). Both HRI and dsI phosphorylate eIF-2α at the same site. However, dsI is induced by interferon and represents an interferon mediated response to viral infection. Since HRI and dsI are eIF-2α kinases, they are both anti-viral in nature, but mechanisms of inactivating dsI by viruses should not affect HRI activity. Therefore, when introduced into the proper target, HRI should be as potent or more potent than dsI in its anti-viral action.

Deletion mutants of HRI cDNA can be constructed that are insensitive to regulation by heme. This heme-insensitive HRI should be more effective than native HRI in its anti-viral and anti-proliferative action.

## Brief Description of the Drawings

Figure 1 is a schematic of eukaryotic initiation of protein synthesis. Numbers in circles refer to eukaryotic initiation factors.

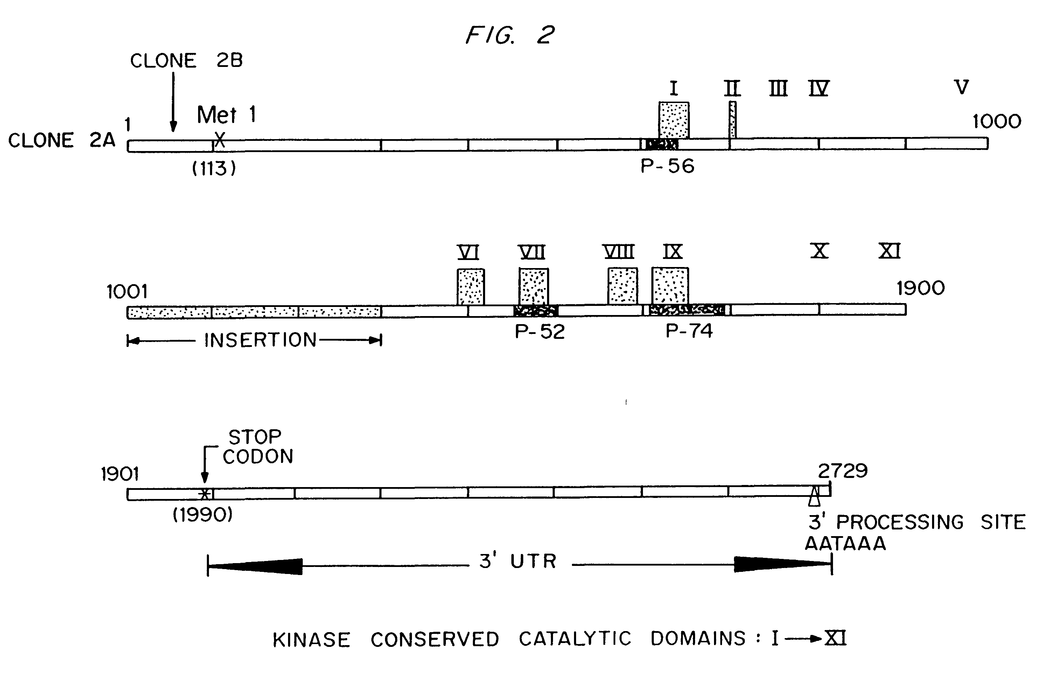

Figure 2 is a schematic of HRI cDNA indicating the locations of the eleven domains, the HRI specific insertion region, and the three peptides previously sequenced and identified as unique to HRI: P-52, corresponding to amino acids 454 to 467, containing Asp-Phe-Gly, which is the most highly conserved short stretch in catalytic domain VII of protein kinases; P-74, corresponding to amino acids 506 to 525, containing the conserved amino acid residues Asp-(Met)-Tyr-Ser-(Val)-Gly-Val found in catalytic domain IX of protein kinases, and P-56, corresponding to amino acids 166-178.

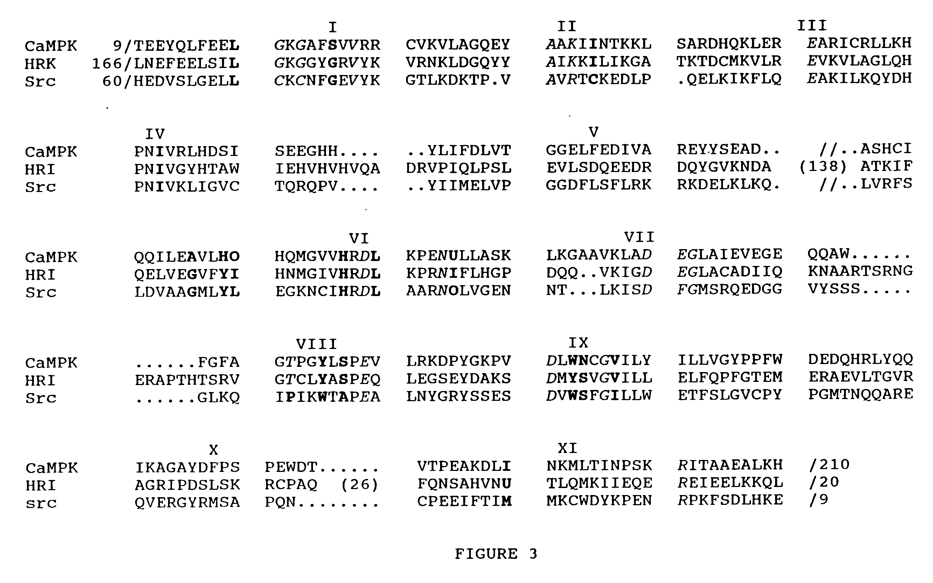

Figure 3 is an alignment of the conserved catalytic domains of HRI with other protein kinases. The conserved catalytic domains are indicated by the Roman numerals (I to XI). The conserved invariant amino acid residues are shown in italicized letters. The semi-conserved amino acid residues of similar structure are shown in bolded letters. Small gaps are shown by dots (····). There is an insertion of 138 amino acids in 'HRI between domains V and VI as indicated by {138}. There is an insertion of 26 amino acids in HRI between domains X and XI as indicated by {26}. The additional amino acids beyond the conserved domains are indicated by the numbers on both N- and C-termini. Single letter code of amino acids is used.

Figures 4A and B are dot-matrix analyses showing homology. (A) Dot-Matrix analysis of the amino acid sequences of HRI and GCN2. (B) Dot-Matrix analysis of the amino acid sequences of HRI and dsI. The dot-matrix was performed using Compare program of Maizel, J. V., Jr. and Lenk, R. P., *Proc. Natl. Acad. Sci.,* USA 78:7665-7669 (1981), with window of 30 and stringency of 15. The locations of the conserved catalytic domains of protein kinases are indicated.

Figure 5A is a photograph of a Northern blot showing expression of HRI mRNA in mouse erythroid cells, using as the probe rabbit HRI cDNA from nucleotides 113 to 2149 (all HRI coding sequences and 159 nucleotides of 3' non-coding sequence).

Figure 5B is a photograph of a Northern blot showing expression of HRI mRNA in human erythroid cells, using the same probe as in Figure 5A.

Figure 6 is a photograph of an agarose gel showing amplification of a human HRI cDNA sequence using the rabbit HRI cDNA sequence: lanes 1-3, primers were nucleotides 229-249 and 543-560; lanes 4-6, primers were nucleotides 448-468 and 1009-1031.

## Detailed Description of the Invention

HRI cDNA was cloned from a lambda Zap II cDNA library of rabbit reticulocytes. As described in more detail below, this cDNA is highly homologous to human DNA encoding HRI and has been used to obtain a clone encoding the human HRI, as well as HRI from other species such as mouse (although there appears to be slightly greater homology between rabbit and human than between rabbit and mouse HRI). The rabbit HRI cDNA contains 2729 nucleotides and encodes 626 amino acids. The nucleic acid sequence has been deposited in the Gene Bank data base (accession No. M69035). *In vitro* translation of HRI mRNA transcribed from HRI cDNA yields a 90 kDa polypeptide with eIF-2$\alpha$ kinase activity. This 90 kDa polypeptide is recognized by anti-HRI monoclonal antibody. These properties are characteristic of authentic HRI.

The open reading frame sequence of the HRI cDNA contains all eleven catalytic domains of protein kinases with consensus sequences of serine/threonine protein kinases in conserved catalytic domains VI and VIII. The HRI cDNA also contains an insert of approximately 140 amino acids between catalytic domains V and VI. The HRI cDNA coding sequence has extensive homology to GCN2 protein kinase of *S. cerevisiae* and to human double stranded RNA-dependent eIF-2$\alpha$ kinase. It therefore is believed that GCN2 protein kinase may be an eIF-2$\alpha$ kinase in yeast. Recently, it has been shown that phosphorylation of e2F-2$\alpha$ by GCN2 is required for the translational control of yeast GCN4, Dever, et al., Cell 28, 585-596 (1992).In addition, HRI has an unusually high degree of homology to three protein kinases, Nim A, Weel and CDC2, which are involved in the regulation of the cell cycle.

## Isolation and sequencing of cDNA encoding HRI from rabbit reticulocytes.

PCR Amplification of HRI cDNA between P-52 and P-74.

Poly A$^+$ mRNA (1 $\mu$g) was reverse-transcribed to obtain single stranded cDNAs according to the method of Frohman, M. A., Dush, M. V. and Martin, G. R., (1988) *Proc. Natl. Acad. Sci.* USA, 85, 8998-9002. The sense-strand oligo-deoxynucleotide of P-52 and the antisense-strand oligo-deoxynucleotide of P-74, deduced with preferred codon usage as in Lathe, R., (1985) J. Mol. Biol., 183, 1-12, were used as primers. The PCR reactions were carried out in the presence of single stranded cDNA template and each primer (1 $\mu$M) for 40 cycles (94°C x 1 min, 47°C x 2 min and 72°C x 3 min).

Preparation of lambda Zap II cDNA library of rabbit reticulocytes and the isolation of HRI cDNA clones.

cDNAs of rabbit reticulocytes were prepared using Pharmacia's cDNA synthesis kit. The cDNAs larger than 500 bp were pooled and were ligated to lambda Zap II vector (Stratagene). The cDNA library obtained has 95% recombinant efficiency. The cDNA library was hybridized at 42°C overnight in a solution containing 5X Denhardt's solution, 6X SSPE, salmon sperm DNA (500 mg/ml), tRNA (1.7 mg/ml), 0.4% SDS plus heat-denatured nick-translated [$^{32}$P] -HRI cDNA probe (10$^6$ cpm/ml). (1 X SSPE = 0.18 M NaCl/10 mM Na phosphate/1 mM EDTA pH 7.4; 1 X Denhardt's solution = 0.02% polyvinylpyrrolidone/0.02% Ficoll/0.02% BSA). The nitrocellulose was then washed three times with 6X SSPE and 0.1% SDS at room temperature for 5 minutes each, followed by washing twice at 50°C under the same salt conditions for 10 minutes each. HRI cDNA was subcloned into pBlue Script plasmid by *in vivo* excision from the recombinant lambda Zap II as described by Stratagene. The DNA sequence of HRI cDNA was determined by the method of dideoxynucleotide chain termination of Sanger, et al., *Proc. Natl. Acad. Sci.,* USA, 74:5463-5467 (1977) with the modification described by Fawcett and Barlett, *BioTechniques,* 9:46-48 (1990).

It was not possible to use P-52, P-56, or P-74 alone to screen the library. In fact, only one of the oligonucleotides derived from these peptides worked in a Northern blot. It was necessary to combine two of the peptides in one of the many possible orientations in order to develop a probe that was useful in pulling out a full length clone.

The homology of the amino acid sequences of HRI tryptic peptides P-52 and P-74 to the conserved domains VII and IX of protein kinases made it possible to predict that P-52 was positioned to the N-terminal side of P-74. This information was used to design primers for PCR amplification of a partial HRI cDNA. Using these two primers, two amplified cDNA fragments which were approximately 230 bp in length were obtained.

This cDNA fragment was subcloned and sequenced. Excluding the 15 bp *Eco*R1 restriction sites present on both primers, the remaining 219 bp sequence encodes an open reading frame for 73 amino acids. The newly obtained 38 amino acid sequence of HRI deduced from this cDNA sequence contains the consensus sequence (Gly-Thr/Ser-X-X-Tyr/Phe-X-Ala/Ser-Pro-Glu) of serine/threonine protein kinases located in the conserved domain VIII. This observation is consistent with the finding by Pathak, et al., *Mol. Cell. Biol.*, 8:993-995 (1988), that HRI phosphorylates eIF-$2\alpha$ at serine -51. Furthermore, the amino acid sequences of HRI between conserved domains VII, VIII, and IX are unique to HRI.

150,000 recombinant clones were screened with the 234 bp probe of HRI. Among the 12 positive clones of the primary screen, five were full-length and contain a cDNA insert of approximately 2700 bp. The 2729 nucleotide sequence of HRI cDNA is shown below. There are 112 nucleotides preceding the first ATG. Starting from this first ATG (nt 113), the open reading frame continues to nucleotide 1990 encoding 626 amino acids followed by multiple stop codons in the 3' untranslated region of 739 nucleotides. It should be noted that the first 250 nt of HRI cDNA are very rich in GC content (80%). The nucleotide sequence of this area of HRI cDNA was finally obtained by using terminal deoxytransferase and pyrophosphatase. The overlapping repeat of the AATAAA polyadenylation signal is found at nucleotides 2689-2698, 11 nucleotides from the poly A tail. The deduced amino acid sequence of the HRI cDNA contains the exact amino acid sequences of the three tryptic peptides of HRI previously obtained by microsequencing. P-52 is located in domain VII, P-56 in domain I, and P-74 in domain IX.

The deoxynucleotide sequence (Sequence ID No. 1) and deduced amino acid sequence (Sequence ID No. 2) of HRI cDNA are shown as Sequence Listing ID Nos. 1 and 2, respectively.

**Expression and characterization of HRI from the isolated cDNA.**

The 5' untranslated leader sequence of the HRI cDNA was replaced by the use of PCR to introduce a unique *Nco*I site (CCATGG) at the initiating methionine (nt 113), followed by ligation of the coding sequence to a vector containing the tobacco mosaic virus (TMV) untranslated leader sequence which was engineered to provide both the initiating methionine and 3'-terminal *Nco*1 site. The introduction of the *Nco*I site changes the second amino acid of HRI from leucine to valine, constituting a conservative substitution.

Linearized HRI cDNAs were transcribed using T7 polymerase. *In vitro* translation of HRI mRNA (40 $\mu$g/ml) was carried out in the presence of [$^{35}$S]-methionine as described by Promega using nuclease-treated reticulocyte lysates or wheat-germ extracts. Protein kinase assays were carried out in 40 $\mu$l reactions with 10 mCi of [g-$^{32}$P]ATP (3,000 Ci/mmol), 1.5 $\mu$l of translational mixture and purified rabbit eIF-2 (1 $\mu$g) as indicated, at 30°C (reticulocyte lysate) or 25°C (wheat germ extract) for 10 min as described by Chen, J.-J., et al., *J. Biol. Chem.,* 264:9559-9564 (1989).

*In vitro* transcription and translation were carried out in order to determine the apparent molecular size of the protein encoded by the HRI cDNA and to test for protein kinase activity. Translation of all five HRI clone mRNAs in a nuclease-treated rabbit reticulocyte lysate yielded a predominant 90 kDa product as observed by SDS-PAGE.

The nucleotide sequence data demonstrate that the 5' untranslated leader sequence is extremely G-C rich with the potential to form significant secondary structure. Secondary structure at the 5'-terminus of mRNAs is known to diminish mRNA translational efficiency. The HRI mRNA was not translatable in a wheat germ extract. Unlike the reticulocyte lysate, the wheat germ extract does not contain an endogenous HRI enzyme; therefore, expression of the HRI protein in the wheat germ system should facilitate analysis of kinase activity in the HRI translation products. The translational efficiency of mRNA transcripts can be increased by the use of untranslated leader sequences of some plant viral RNAs such as TMV have been shown to act in cis by Gallie, et al., (1987) *Nucl. Acids Res.,* 15, 8693-8711, and Gehrke, L. and Jobling, S. A., (1990) In: McCarthy, JEG *Post-Transcriptional Regulation of* Gene *Expression, Series H: Cell Biology,* ed. Tuite, M. (Springer Verlag, Berlin), Vol. 49, pp. 389-398. Accordingly, the G-C rich HRI untranslated leader sequence was replaced with that of TMV. The chimeric TMV-HRI mRNA was translated with approximately tenfold greater efficiency than HRI mRNA in the reticulocyte lysate, and translation in the wheat germ extract was clearly evident. In all cases, the translated product of HRI mRNA migrated slightly faster than authentic purified phosphorylated HRI on SDS gel electrophoresis. This slight difference in mobility is most likely due to a lower level of phosphorylation in the translation products.

To determine whether the translational product derived from the mRNA of HRI cDNA is an eIF-$2\alpha$ kinase, a small portion of the total translation mixture was incubated with purified rabbit reticulocyte eIF-2 and [g-$^{32}$P] ATP in the absence of added hemin under protein kinase assay conditions and analyzed by SDS-gel electrophoresis.

The results show that translational products of HRI 2A and HRI 2B mRNAs have enhanced eIF-$2\alpha$ kinase

activity as compared to the control in the absence of added mRNA. It should be emphasized that under the kinase assay conditions (final hemin concentration of 0.75 μM) the activity of newly synthesized HRI exceeds the low activity of endogenous pre-formed HRI in the nuclease-treated lysate and makes it possible to detect enhanced phosphorylation of eIF-2α. In the absence of added purified rabbit eIF-2, only slight phosphorylation in the region of eIF-2α is observed. Furthermore, the HRI polypeptide synthesized in the wheat-germ extracts exhibits eIF-2α kinase activity as does purified HRI. It should be noted that there is no mammalian eIF-2α kinase activity in the wheat-germ extracts, and the purified reticulocyte HRI phosphorylates purified wheat germ eIF-2α very inefficiently. In addition, the 90 kDa polypeptide expressed from HRI cDNA is immunoprecipitated by monoclonal antibodies to HRI.

**Isolation of cDNA encoding HRI in other mammalian species.**

DNA nucleotide sequence data were analyzed in part using CAD Gene™ software for the MacIntosh™ computer, provided by the Genetic Technology Corporation, Cambridge, MA. The amino acid sequences of dsRNA-dependent eIF-2α kinase (dsI) of rabbit and human are 83% similar and 76% in identity. Similar or higher degree of homology of initiation factors (eIF-2α, and eIF-2β eIF-4A, eIF-4E, EF-1a) between human and rabbit has been demonstrated. The predicted homology of HRI between human and rabbit is greater than 80%. Accordingly, the sequence encoding HRI in human or other species can be isolated by hybridization under standard conditions such as those outlined by Maniatis, et al., (1989) *Molecular Cloning. A Laboratory Manual,* from a library prepared from reticulocytes of the other species. The isolated sequence can then be expressed in the same manner as the HRI cDNA isolated from rabbit reticulocytes as described below in the Examples.

**High degree of homology between HRI and other protein kinases.**

Purified non-recombinant HRI undergoes heme-regulated autophosphorylation and eIF-2α phosphorylation. The sites of autophosphorylation of many protein kinases are located within 20 amino acids of the conserved Ala/Ser-Pro-Glu sequence in catalytic domain VIII (e.g. Thr-197 of cAMP-dependent protein kinase). The HRI-equivalent of the Thr-197 of cAMP-dependent protein kinase is Thr-483. In addition, there are two serine and three more threonine residues in the vicinity of Thr-483. Since HRI can undergo multiple phosphorylation *in vitro* the availability of HRI cDNA will facilitate the further study of the sites and role of autophosphorylation in the activation of HRI.

Comparison of HRI and dsI and GCN2 protein kinase.

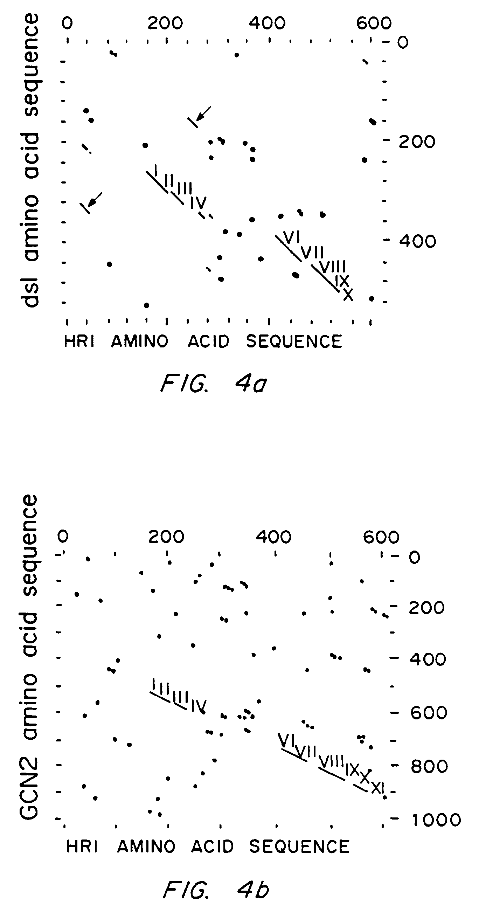

Comparison of the amino acid sequences of HRI and dsI deduced from the cDNAs indicates that in addition to general homology in kinase conserved domains, there is a very significant homology of both eIF-2α kinases around domains IX and X (HRI amino acid 511-540), as shown in Figure 4A. It is likely that these amino acids are involved in eIF-2 binding and the phosphorylation of eIF-2α. In addition, HRI synthetic peptide P-74 which resides around domain IX inhibits the eIF-2α kinase activity of both HRI and dsI.

The Gene Bank has been searched for homology to other protein sequences of the amino acid sequence of HRI deduced from its cDNA. Of the ten proteins with the highest scores (Table I), nine are Ser/Thr protein kinases, and of these, three are involved in regulation of the cell cycle (Nim A, Wee1 and CDC2).

It is especially noteworthy that GCN2 protein kinase of yeast displays more homology to HRI than does dsI, the other known eIF-2α kinase (Table I). The scores of homology of HRI to GCN2 and dsI are significantly higher than those to other protein kinases (Table I). The cDNA of human dsI was recently cloned by Meurs, et al., (1990) *Cell,* 62: 379-390. A dot-matrix homology analysis of HRI and dsI coding sequences is shown in Fig. 4A, and a similar analysis of HRI and the kinase moiety of GCN2 coding sequences is shown in Fig. 4B. These dot-matrix plots reveal the extensive homology of these three proteins in the protein kinase catalytic domains I through X except for domain V where HRI has a large kinase insertion sequence. Homology in domains IX and X is observed only with HRI, dsI and GCN2, but not with the other eight protein kinases with the best scores. The significant homology in these regions suggests that these amino acids may be involved in the binding and phosphorylation of eIF-2, and raise the possibility that GCN2 protein kinase may be an eIF-2α kinase in yeast.

Table 1.

| Homology of HRI to other Protein Kinases. A score of 300 to 400 indicates approximately 50% homology, whereas a score of 200 indicates approximately 25% homology. | |
| --- | --- |
| **Kinase** | **Scores** |
| GCN2 protein kinase (Yeast) | 383 |

Table 1.   (continued)

| Homology of HRI to other Protein Kinases. A score of 300 to 400 indicates approximately 50% homology, whereas a score of 200 indicates approximately 25% homology. | |
| --- | --- |
| **Kinase** | **Scores** |
| dsRNA-dependent eIF-2$\alpha$ kinase (Human) | 331 |
| Ca$^{+2}$/calmodulin protein kinase (Rat) | 252 |
| Never-in-Mitosis gene product (Yeast) | 249 |
| Wee 1 gene product (Yeast) | 246 |
| Type II Ca$^{+2}$/calmod kinase (Rat brain) | 222 |
| Calmodulin-dependent protein kinase (Rat) | 211 |
| Calmodulin-dependent protein kinase II (Rat) | 211 |
| M38724 Mus musculus cell cycle protein | 209 |
| Calmodulin-dependent protein kinase II (Rat) | 207 |
| CDC2 gene product (Human) | 206 |
| cAMP-dependent protein kinase (Yeast) | 205 |
| Protein kinase gene (Yeast) | 205 |
| M37712 p58/GTA protein kinase (Human) | 204 |
| cAMP-dependent kinase (Yeast) | 197 |
| TPK2 gene (Yeast) | 195 |
| cAMP-dependent kinase | 194 |
| Protein Kinase C (Rat) | 192 |
| Protein kinase C zeta-subspecies (Rat) | 184 |
| CDC2 cell division gene (Yeast) | 184 |
| Varicella-Zoster virus complete genome | 181 |
| Muscle light chain kinase (Rat) | 180 |
| HSV-2 genomic HindIII 1 region | 180 |
| Tyrosine kinase (Rat) | 180 |
| Src (Rat) | 180 |
| Tyrosine kinase (Human) | 176 |
| The homology of the protein sequence of HRI to those of other proteins in Gene Bank was determined using Fast A program of Pearson, W. R. and Lipman, D. J., (1988) *Proc. Natl. Acad. Sci.,* USA, 85:2444-2448. | |

GCN2 protein kinase of yeast displays very significant homology to HRI (Table I and Figure 4B) especially in domains IX and X in which considerable homology is observed only in eIF-2$\alpha$ kinases. GCN2 protein kinase stimulates the expression of amino acid biosynthetic genes under conditions of amino acid starvation by derepressing GCN4, a transcriptional activator of these genes. The derepression of GCN4 by GCN2 protein kinase occurs at the level of translation of GCN4 mRNA. The activation of the translation of GCN4 mRNA coincides with a decrease in the rate of general polypeptide chain initiation at the level of eIF-2 dependent 43 S pre-initiation complex formation. Furthermore, a yeast strain that overexpresses GCN2 protein kinase has been reported to have a lower rate of protein synthesis. Thus, the effect of GCN2 protein kinase on protein synthesis is very similar to that of HRI. The molecular cloning of yeast eIF-2$\alpha$ by Cigan, et al., (1989) *Proc. Natl. Acad. Sci*., USA, 86:2784-2788, reveals 58% homology of its amino acid sequence to human eIF-2$\alpha$, as reported by Ernst, et al., (1987) *J. Biol. Chem.,* 262:1206-1212. In addition, consensus phosphorylation site Ser-51 is conserved in yeast eIF-2$\alpha$, and the phosphorylation of yeast eIF-2$\alpha$ has been demonstrated by Cigan, et al. The possibility that GCN2 protein kinase may phosphorylate eIF-2 has been raised by Cigan et al and Tzamarias et al, (1989) *Cell,* 57:947-954. The alignment of the amino acid sequences of HRI and GCN2 indicates 52% similarity and 28% identity in the kinase moiety of GCN2. This extensive homology of HRI and GCN2 affords further support for the view that GCN2 may be an eIF-2$\alpha$ kinase in yeast. Recently, it GCN2 may be an eIF-2$\alpha$ kinase in yeast. Recently, it has been demonstrated that phosphorylation of e2F-2$\alpha$ is required for the translational control of yeast GCN4, Dever, et al., Cell 68, 585-596 (1992).

Comparison of unique insertion sequence.

As shown in Figure 4, HRI cDNA contains an insertion of approximately 140 amino acids between catalytic domains V and VI (amino acids 276 to 413). Similar large inserts have been reported for subclass III and IV receptor tyrosine kinases, which include the PDGF receptor, the CSF-1 receptor and the c-kit proto oncogene product, in which the kinase domains are divided into two halves by insertion of up to 100 mostly hydrophilic amino acid residues, as reviewed in Ullrich, A. and Schlessing, J., (1990) *Cell,* 61:203-212. Since kinase insertion sequences are highly conserved among species for each specific receptor, the kinase insert may play an important role in the action of receptor kinases. Indeed, the PDGF receptor kinase insert contains an autophosphorylation site (Tyr-751), and mutation of Tyr-751 to Phe or Gly blocks association of the PDGF receptor with phosphatidylinositol kinase and three other cellular proteins. In the case of HRI, heme binds to HRI and regulates its kinase activities. It is believed that the kinase insertion sequence of HRI is involved in the binding of heme and the regulation of the autokinase and eIF-2$\alpha$ kinase activities.

Comparison of HRI and protein kinases involved in the cell cycle.

There is also a high degree of homology between HRI and several protein kinases involved in the cell cycle.

Hanks, Quinn and Hunter (1988) *Science,* 241:42-52, have compared and aligned the protein sequences of 65 different protein kinases. They have identified eleven domains of protein kinases with alignment of the HRI sequence with the sequences of a serine/threonine protein kinase (Ca$^{++}$/calmodulin protein kinase) and of a tyrosine protein kinase (Src) is shown in Figure 3. HRI cDNA contains all eleven catalytic domains with invariant amino acid residues, as also shown in Figure 4. The consensus ATP-binding sequence, Gly-X-Gly-X-X-Gly, and the invariant valine residue located two positions downstream of the Gly-X-Gly-X-X-Gly are conserved in HRI. In domain II, the invariant Lys residue has been shown to be indispensable and to be involved in the phosphotransferase activity of protein kinases. In HRI this invariant residue is Lys-199. Domain VI contains the consensus sequence which specifies either Ser/Thr protein kinases or Tyr protein kinases. HRI possesses Asp-Leu-Lys-Pro-Arg-Asn in domain VI which is characteristic of Ser/Thr protein kinases. Asp-Phe-Gly located in domain VII is the most conserved short stretch in the catalytic domains of protein kinases and is probably involved in ATP-binding. It is found in HRI as Asp(-456)-Phe(-457)-Gly(-458). In domain VIII the Ala/Ser-Pro-Glu consensus sequence essential for catalytic activity of protein kinases is also found in HRI. Domain VIII of HRI contains the other consensus sequence for Ser/Thr protein kinases, Gly-Thr-Cys-Leu-Tyr. The conserved amino acids in domain IX are also found in HRI. Thus, the homology of the deduced amino acid sequence of HRI cDNA to the conserved domains of other Ser/Thr protein kinases provides confirmatory evidence that HRI cDNA encodes a Ser/Thr protein kinase.

**Inhibition of Cell Proliferation and Differentiation and viral activity and the induction of Differentiation using HRI or dsI.**

Since HRI is a potent inhibitor of protein synthesis, it is anti-proliferative in nature and should be useful in the treatment of various cancers in which uncontrolled cell growth persists, for example chronic myelogenous leukemia. HRI should also be useful in treatment of other proliferative disorders such as psoriasis.

Initiation of protein synthesis can also be regulated by another eIF-2$\alpha$ kinase which is activated by double-stranded RNA (dsI). Both HRI and dsI phosphorylate eIF-2$\alpha$ at the same site. However, HRI and dsI are different molecules. dsI is induced by interferon and represents an interferon mediated response to viral infection. However, mechanisms of inactivating dsI have evolved in various viruses to undermine the anti-viral action of dsI. Since HRI and dsI are both eIF-2$\alpha$ kinases, both should be anti-viral in nature. However, mechanisms of inactivating viruses by dsI should not similarly affect HRI activity. Therefore, when introduced into the proper target cell, HRI may be as potent or more potent than dsI as an anti-viral agent.

Based on the similarity to proteins involved in cellular differentiation, it is expected that HRI will induce differentiation. As noted above, CDC2, Wee 1 and Nim A contain consensus sequences for serine/threonine protein kinases. They were identified first in yeast by genetic means. However, CDC2 and Wee 1 have also been isolated and characterized in human cells. The CDC2 gene product is required for both S phase and mitosis. The Wee 1 gene product is an inhibitor of mitosis. Nim A gene product is an activator of mitosis. Both Nim A and Wee 1 gene products regulate the cell cycle through regulation of CDC2 kinase which is part of the mitosis promoting factor. Many types of cancers are characterized by arrested differentiation. HRI can also be introduced into these cells to induce differentiation and thereby limit the proliferation of the transformed cells.

Since HRI is expressed normally only in very small quantities, in the cytoplasm, and during specific periods of erythroid differentiation, small quantities of the protein are expected to be effective in inhibiting protein synthesis, inducing differentiation, and inhibiting infection by viruses and parasites.

The HRI, expressed from the cDNA, preferably of the same species as the cells to be treated, can be administered topically, by injection, or via implant to the cells or patient to be treated. Appropriate pharmaceutical compositions and methods for administration and use thereof are well known to those skilled in the art. The HRI can be expressed in any suitable mammalian expression system, using known technology, under the control of appropriate enhancers and promoters.

Alternatively, the cells to be treated are "infected" with the sequence encoding the HRI. In the preferred embodiment, this is accomplished by inserting the HRI sequence into a retroviral vector with which the cell is then infected. For example, a retroviral vector for gene transfer and expression of HRI cDNA can be constructed using as the backbone of the retroviral vector the LNCX vector described by Miller and Rosman (Miller, A.D., and Rosman, G.J. (1989) *Bio-Techniques* 7:980-990). It contains human cytomegalovirus (CMV) immediate early gene promoter and enhancer. HRI cDNA containing TMV-leader sequence is introduced into the LNCX vector through a polylinker region downstream from the CMV promoter.

```
LNCX        LTR----------NEO|        |CMV-----LTR
                                                    ↑
                        TMV-HRI cDNA
```

Gene transfer by retroviral vector can also be achieved by transfection by a viral vector, using the method of Wilson, J.M, Biriuyi, L.U., Salomon, R.U., et al. Transplantation of Vascular Grafts Lined With Genetically Modified Endothelial Cells. *Science,* 244:1344-1346 (1980), or a plasmid transfer technique, as described by Felgner, P.L., Galik, T.R., Holmer, et al. Lipofection: An Efficient, Lipid Mediated DNA-Transfection Procedures. *Proc. Natl. Acad. Sci.,* 84: 7413-7417 (1987), the teachings of which are incorporated herein by reference.

Specifically, cells are harvested, grown to subconfluence (60-70%) and incubated with a replication defective murine Moloney leukemia retroviral vector. The DNA sequence for HRI is inserted into the viral genome and is under the promoter control of the viral long-terminal repeats (LTR's). The infected cells are trypsinized, resuspended in saline containing penicillin (100 U/ml) and streptomycin (100 µg/ml) and transplanted into the patient requiring treatment. The presence of HRI in the culture medium or the site of transplantation can be determined by radioimmunoassay.

**Construction of deletion mutants of HRI cDNA that are insensitive to heme, less species specific or overexpressed.**

Deletion mutants of HRI cDNA which are not sensitive to regulation by heme can be constructed, based on the prediction that the heme-binding region is found within the HRI-specific insert discussed above and/or in the 170 N-terminal amino acids. This heme-insensitive HRI may be more effective than native HRI in its anti-viral and anti-proliferative action.

Deletion mutants of HRI cDNA can also be constructed which are less species specific. There is greater than 80% homology between species (86% between human and rabbit dsI). The primary area of species variation is in domain V. Methods for constructing and screening for these mutations are known to those skilled in the art.

**Example 1: Expression of HRI mRNA in Human and Mouse Erythroid Cells.**

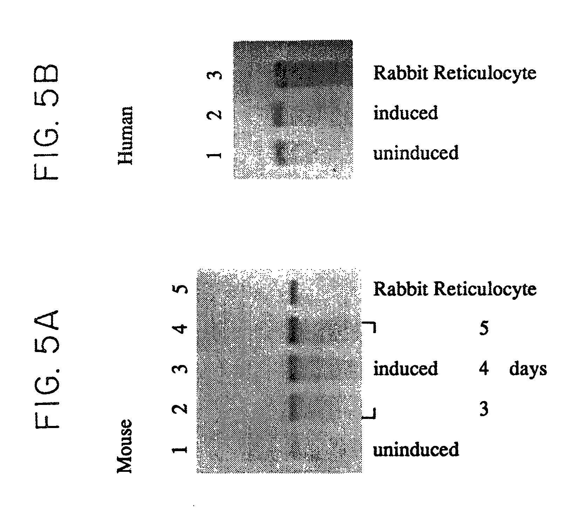

The expression of HRI mRNA in human erythroleukemia cells (K562) and mouse Friend erythroleukemia cells (MEL) during erythroid differentiation was examined by Northern-Blot analysis. The probe used for the Northern-Blot analysis shown in Figs. 5A and 5B is the above-described rabbit HRI cDNA from nucleotides 113 to 2149 which is comprised of all the coding sequences of HRI cDNA and 159 nucleotides of the 3' non-coding sequence. The results in Figs. 5A and 5B show that rabbit HRI cDNA hybridizes to a 3.1 Kb mRNA from both MEL (Panel A) and K562 (Panel B) cells. The HRI mRNA from K562 and MEL cells appears to be of the same size as rabbit HRI mRNA. In addition, HRI mRNA is increased upon erythroid differentiation induced by incubation of cells with hemin for four days.

Cell Culture

K562 cells were maintained in RPMI 1640 medium containing 10% fetal calf serum and antibiotics at 37°C in 5% $CO_2$. Cells were treated with 75 mM hemin by dilution of a 1 mM hemin stock solution directly into the culture medium. After four days, the cells were washed twice in culture medium and incubated in their normal growth medium for 12 hours. The cells were then collected and washed twice in phosphate buffered saline (PBS). MEL cells were grown in Dulbeco modified minimum essential medium containing 10% fetal calf serum. Cells were induced for erythroid differentiation by the addition of 2% dimethylsulfoxide (DMSO). Cells were harvested 3, 4 and 5 days after DMSO-treatment as described above.

mRNA Isolation and Northern Blotting

Poly (A)$^+$ mRNA was isolated from both untreated K562 cells, hemin treated K562 cells, untreated MEL cells and DMSO-treated MEL cells using oligo (dT) cellulose and an Invitrogen™ mRNA Isolation Kit. 1 x 10$^8$ cells consistently yielded 15-20 μg of high quality mRNA. 5 μg of each sample mRNA were denatured and separated on 1.0% agarose formaldehyde denaturing gels. mRNA was transferred to a nitrocellulose membrane in 20X SSPE overnight at room temperature and cross-linked to the nitrocellulose by UV irradiation.

Hybridization

Nitrocellulose filters were prehybridized in 50% formaminde, 6X SSPE, 5X Denhardt's solution, 0.5% SDS, 100 mg/ml salmon sperm DNA and 10% dextran sulfate, at 42°C for overnight (12-14 hrs). Hybridization took place under the same conditions but for the addition of 1-3 x 10$^9$ cpm/μg $^{32}$P-labeled HRI cDNA. Nitrocellulose filters were washed 3 times for 5 minutes in 2X SSPE + 0.1% SDS at room temperature (R.T.), followed by washes of 1X SSPE + 0.1% SDS for 10 minutes at room temperature, 1X SSPE + 0.1% SDS for 10 minutes at 50°C, and 0.2X SSPE + 0.1% SDS for 10 minutes, 50°C. Membranes were exposed to film (Kodak X-AR) at -80°C with an intensifying screen.

**Example 2: Amplification of Human HRI cDNA Sequence Using Rabbit HRI cDNA Sequence.**

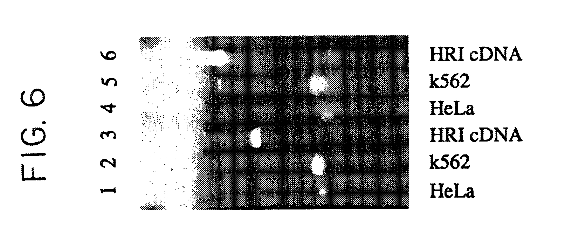

Several oligonucleotides of the above-described rabbit HRI cDNA were used as primers for the polymerase chain reaction (PCR) to amplify the human HRI cDNA sequence. The poly A$^+$ mRNAs from hemin-induced K562 cells was reversed-transcribed to obtain single-stranded cDNAs. This single-stranded cDNA preparation was used as a template to amplify a human HRI cDNA sequence. The primers used for PCR reactions shown in Lanes 1 to 3 of Fig. 6 are nucleotides 229-249 and nucleotides 543-560. An expected DNA fragment of 331 bp was amplified from cloned HRI cDNA 2B of rabbit (Lane 3). A faint but detectable DNA fragment of the same size was also amplified from the cDNA reversed-transcribed from human K562 poly A$^+$ mRNA (Lane 2), but not from human HeLa cells (Lane 1). The primers used for PCR reactions shown in Lanes 4 to 6 are nucleotides 448-468 and nucleotides 1009 to 1031. An expected DNA fragment of 584 bp was amplified from cloned rabbit HRI cDNA 2B (Lane 6) and from human K562 cDNA (Lane 5). These two amplified human HRI cDNA fragments are located in the N-terminus of HRI coding sequence where conserved sequences of protein kinase are not found. The lack of homology of the N-terminus of HRI to other nucleotide sequences is shown in Table 2. The N-terminus of protein kinases is usually devoted to a regulatory role of a particular protein kinase. The result of a search of the GeneBank database indicates that the first 170 amino acids of the N-terminus of HRI is unique to HRI; no significant homology to other eIF-2α kinases (GCN2 and dsI) is observed. Therefore, the amplification of human HRI cDNA sequences from rabbit HRI cDNA shown in Fig. 6 is very significant and demonstrates the extensive homology of human HRI cDNA to rabbit HRI cDNA. The human HRI cDNA sequence in the conserved domains from domain VII to domain IX was also amplified.

Table 2.

| Non-Homology of N-terminus of HRI to other Kinases. A score of 300 to 400 indicates approximately 50% homology, whereas a score of 200 indicates approximately 25% homology. | |
| --- | --- |
| **Kinase** | **Scores** |
| Varicella-Zoster virus complete genome | 100 |
| Skeletal muscle voltage-sensitive Na+ channel (Rat) | 96 |
| Cytomegalovirus (HCMV) (Human) | 92 |
| Foot and Mouth Disease Virus | 92 |
| Adenylate cyclase gene | 90 |
| cGMP-dependent protein kinase (D.melanogaster) | 88 |
| Mesothelial keratin K7 (type II) (Human) | 88 |
| Mei2 gene (Human) | 86 |
| The non-homology of the protein sequence of the N-terminal of HRI to other sequences in Gene Bank was determined using Fast A program of Pearson, W. R. and Lipman, D. J., (1988) *Proc. Natl. Acad. Sci.,* USA, 85: 2444-2448. | |

The results of Figs. 5A, 5B and 6 demonstrate that HRI cDNA from species other than rabbits, including humans, can be cloned using rabbit HRI cDNA from a cDNA library of hemin-treated K562 cells.

Modifications and variations of the present invention, the cDNA encoding HRI, and methods of use thereof, will be obvious to those skilled in the art from the foregoing detailed description. Such modifications and variations are intended to come within the scope of the following claims.

SEQUENCE LISTING

(1) GENERAL INFORMATION:

    (i) APPLICANT: Massachusetts Institute Of Technology

    (ii) TITLE OF INVENTION: DNA Encoding the Heme-Regulated Eukaryotic Initiation Factor 2 alpha Kinase

    (iii) NUMBER OF SEQUENCES: 2

    (iv) CORRESPONDENCE ADDRESS:

        (A) ADDRESSEE: Kilpatrick & Cody
        (B) STREET: 1100 Peachtree Street
        (C) CITY: Atlanta
        (D) STATE: Georgia
        (E) COUNTRY: US
        (F) ZIP: 30309

    (v) COMPUTER READABLE FORM:

        (A) MEDIUM TYPE: Floppy disk
        (B) COMPUTER: IBM PC compatible
        (C) OPERATING SYSTEM: PC-DOS/MS-DOS
        (D) SOFTWARE: PatentIn Release #1.0, Version #1.25

    (vi) CURRENT APPLICATION DATA:

        (A) APPLICATION NUMBER: US
        (B) FILING DATE:
        (C) CLASSIFICATION:

    (viii) ATTORNEY/AGENT INFORMATION:

        (A) NAME: Pabst, Patrea L.
        (B) REGISTRATION NUMBER: 31,284
        (C) REFERENCE/DOCKET NUMBER: MIT 5807

    (ix) TELECOMMUNICATION INFORMATION:

        (A) TELEPHONE: 404-815-6508
        (B) TELEFAX: 404-815-6555

(2) INFORMATION FOR SEQ ID NO:1:

    (i) SEQUENCE CHARACTERISTICS:

        (A) LENGTH: 2729 base pairs
        (B) TYPE: nucleic acid
        (C) STRANDEDNESS: single
        (D) TOPOLOGY: linear

(ii) MOLECULE TYPE: cDNA

(iii) HYPOTHETICAL: NO

(iv) ANTI-SENSE: NO

(v) FRAGMENT TYPE: N-terminal

(vi) ORIGINAL SOURCE:

(A) ORGANISM: Rabbit
(G) CELL TYPE: Reticulocytes

(ix) FEATURE:

(A) NAME/KEY: misc_feature
(B) LOCATION: 113..2149
(D) OTHER INFORMATION: /note= "Expression of HRI mRNA in Human erythroid cells, using as the probe rabbit HRI cDNA from nucleotides 113 to 2149."

(ix) FEATURE:

(A) NAME/KEY: misc_feature
(B) LOCATION: 229..249
(D) OTHER INFORMATION: /note= "Primer used in the amplification of human HRI cDNA sequence using the rabbit HRI cDNA sequence."

(ix) FEATURE:

(A) NAME/KEY: misc_feature
(B) LOCATION: 543..560
(D) OTHER INFORMATION: /note= "Primer used in the amplification of human HRI cDNA sequence using the rabbit HRI cDNA sequence."

(ix) FEATURE:

(A) NAME/KEY: misc_feature
(B) LOCATION: 448..468
(D) OTHER INFORMATION: /note= "Primer used in the amplification of human HRI cDNA sequence using the rabbit HRI cDNA sequence."

(ix) FEATURE:

(A) NAME/KEY: misc_feature
(B) LOCATION: 1009..1031
(D) OTHER INFORMATION: /note= "Primer used in the amplification of a human HRI cDNA sequence using the rabbit HRI cDNA sequence."

(x) PUBLICATION INFORMATION:

(A) AUTHORS: Chen, Jane J.
    London, Irving M.
(B) TITLE: Cloning of the cDNA of the heme-regulated eukaryotic initiation factor 2alpha (eIF-2alpha) kinase of rabbit reticulocytes:
    Homology to yeast GCN2 protein kinase and human double-stranded-RNA-dependent
(C) JOURNAL: Proc. Natl. Acad. Sci. U.S.A.
(D) VOLUME: 88
(F) PAGES: 7729-7733

# EP 0 658 204 B1

(G) DATE: September-1991

(xi) SEQUENCE DESCRIPTION: SEQ ID NO:1:

```
CGCACGGCGC TCGCGACCCG GACGCGCGAG GAGGCGGTCC CGGAGTCGGG GAGCTGGCGG      60
GTGGGCTGTG GTCCCCGCAT TTGCGCGCGC GGGCGCCCGC GCGTGACCGG CGATGCTGGG     120

GGGCAGCGCC GGGACCCGCG GGGGCGAAGC CGAGGGCGAC GGGGCGGGGG CGGTGGGGGC     180
GGTGGCCCCG CCGCCCGCCA TCGACTTCCC CGCTGAGGTG TCGGATCCCA AGTATGACGA     240
GTCGGATGTC CCGGCAGAGC TGCAGGTGCT GAAGGAGCCG CTGCAGCAGC CAGCCTTCCC     300
CTTCGCCGTC GCCAACCAGC TGCTGCTCGT CTCCCTGCTG GAGCACCTGA GTCATGTGCA     360
CGAGCCAAAC CCGCTTCGCT CCAGACAGGT GTTTAAACTG CTCTGTCAGA CCTTCATCAA     420
AATGGGGCTG CTGTCTTCCT TCACCTGCAG CGACGAGTTT AGCTCATTGA GGCTGCATCA     480
CAACAGAGCT ATTACGCATC TGATGAGGTC CGCCAGAGAG AGAGTTCGGC AGGATCCCTG     540
TGCTGATAAT TCTCATATCC AGAAAATCAG GTCGCGAGAA GTTGCCTTGG AAGCACAGAC     600
CTCACGATAC TTGAATGAGT TTGAAGAGCT CTCCATCCTG GGGAAAGGTG GCTATGGCCG     660
AGTGTACAAG GTCAGGAATA AATTAGATGG CCAGTATTAT GCAATTAAAA AAATTCTGAT     720
TAAAGGTGCA ACTAAAACAG ATTGCATGAA GGTATTACGA GAAGTGAAAG TGCTGGCGGG     780
CCTCCAGCAC CCTAATATCG TAGGCTATCA CACCGCGTGG ATAGAGCATG TCCACGTTCA     840
CGTTCAAGCA GACAGAGTTC CGATTCAGTT GCCTTCTCTG GAAGTGCTCT CTGACCAGGA     900
AGAAGACAGA GATCAATATG GTGTTAAAAA TGATGCAAGC AGCAGCTCAT CCATTATTTT     960
CGCTGAGTTC TCCCCAGAAA AAGAAAAATC CTCTGACGAA TGTGCCGTTG AGAGTCAGAA    1020
TAACAAACTG GTGAACTACA CCACCAACTT AGTGGTGAGG GACACCGGTG AGTTTGAATC    1080
GTCCACGGAG CGCCAAGAGA ACGGCTCGAT CGTGGAGCGT CAGCTACTGT TCGGGCATAA    1140
CTCAGACGTA GAAGAGGATT TCACGTCCGC GGAGGAATCT TCTGAGGAAG ACTTAAGCGC    1200
GTTGCGGCAC ACAGAGGTGC AGTACCACCT GATGCTGCAT ATCCAGATGC AGCTGTGCGA    1260
```

13

```
GCTGTCCCTG TGGGACTGGA TCGCCGAGAG GAACAGGCGG AGCCGAGAGT GCGTGGACGA      1320

ATCTGCCTGT CCTTATGTTA TGGTCAGTGT TGCAACAAAA ATTTTTCAAG AACTGGTGGA      1380

AGGTGTGTTT TACATACATA ACATGGGCAT CGTGCACAGA GACCTGAAGC CTAGAAATAT      1440

TTTTCTTCAT GGTCCTGATC AACAAGTGAA AATAGGAGAC TTTGGTCTGG CCTGCGCCGA      1500

CATCATCCAG AAGAATGCGG CCCGGACCAG CAGAAACGGG GAGAGAGCAC CCACACACAC      1560

TTCCCGAGTG GGCACCTGTC TGTACGCCTC GCCCGAGCAG TTGGAAGGAT CGGAGTATGA      1620

TGCCAAGTCA GACATGTACA GCGTCGGCGT GATCCTGCTG GAGCTCTTCC AGCCCTTCGG      1680

GACAGAGATG GAGCGGGCAG AGGTCCTGAC GGGCGTGCGA GCTGGCCGCA TACCCGACTC      1740

CCTCAGTAAG AGGTGCCCGG CGCAGGCCAA GTACGTCCAG CTGCTGACCA GGAGGAACGC      1800

GTCCCAGCGG CCGTCCGCCC TTCAGCTGCT GCAGAGTGAG CTCTTCCAGA ACTCCGCGCA      1860

TGTTAACCTC ACCCTACAGA TGAAGATAAT AGAGCAGGAA AGAGAAATCG AGGAACTCAA      1920

GAAGCAGCTG AGCCTCCTCT CCCAGGCCCG AGGGGTGAGG AGTGACAGGC GAGACGGAGA      1980

GCTCCCTGCC TAGCCGTCAC TCGGCCACGT CACAGGGGAA CGTGGACTTG CACTTGCAGC      2040

AGTCAACTGG AATGGACAAT TTCAAGCCTC CTGAGGTTCA GGCGGCATAA TCCTCACTTG      2100

GAATCACTCA GCCCGCATGA CTCTCCCCTC ATGCTGCTCT TCCCGGAGGT ACCTCCTGGT      2160

GACCTCCTGG TGACTGCTCC CAATTAAACT TACGCTTTTC CCTTTCCTAT TCCGCAAGTC      2220

CCATTCCTGA GCCTCCTACC TAAGCATTAA CTAAATCTTA GGTATCGGTC TCCATTCTTT      2280

CTCCTTTGAA TCCTGGCCAC CTCGCTCCTT TAGAAGCACA CTCACTGCCC CGCCACCACC      2340


CAAGGCCAGG CCTGCACCCT GGCGCAACAG CTGCCAGTCT TAGTCCTTAG CTGCTGCTGC      2400

TGTTGCCAGA GACACCTGCT CCGTTCACTC CCTCCAGGGT GGAAGCTCAG CCTGTGAGCA      2460

GCGCCTCTGC TCTCCCCGGC TGCAGCCCAG CGCCACTCGG GCAGGCTTCA CACGCTCACC      2520

CCAGGTGGCC TCGGAACAGC TGCGACAGCA TCTCCCCGCA CCCTTCTGCC TTCTCAGCAC      2580

TTGGCTCTCC AGCCAGCCTC TCCACTCACT CGTTTTTGTT TCCCGGAGCT GTCTGCCACA      2640

ATGTTGGCAG TCTTCATGGA CTACTGTACG TGATTCTGCT GAATTTTAAA TAAATAAACC      2700

CTGCAAATCA AAAAAAAAAA AAAAAAAAA                                       2729
```

(2) INFORMATION FOR SEQ ID NO:2:

   (i) SEQUENCE CHARACTERISTICS:

      (A) LENGTH: 626 amino acids
      (B) TYPE: amino acid
      (C) STRANDEDNESS: single
      (D) TOPOLOGY: linear

(ii) MOLECULE TYPE: protein

(iii) HYPOTHETICAL: NO

(iv) ANTI-SENSE: NO

(v) FRAGMENT TYPE: N-terminal

(vi) ORIGINAL SOURCE:

    (A) ORGANISM: Rabbit
    (G) CELL TYPE: Reticulocytes

(ix) FEATURE:

    (A) NAME/KEY: Peptide
    (B) LOCATION: 166..170
    (D) OTHER INFORMATION: /label= P-56

(ix) FEATURE:

    (A) NAME/KEY: Peptide
    (B) LOCATION: 454..459
    (D) OTHER INFORMATION: /label= P-52

(ix) FEATURE:

    (A) NAME/KEY: Peptide
    (B) LOCATION: 506..510
    (D) OTHER INFORMATION: /label= P-74

(xi) SEQUENCE DESCRIPTION: SEQ ID NO:2:

```
Met Leu Gly Gly Ser Ala Gly Thr Arg Gly Gly Glu Ala Glu Gly Asp
1               5                   10                  15

Gly Ala Gly Ala Val Gly Ala Val Ala Pro Pro Pro Ala Ile Asp Phe
                20                  25                  30

Pro Ala Glu Val Ser Asp Pro Lys Tyr Asp Glu Ser Asp Val Pro Ala
            35                  40                  45

Glu Leu Gln Val Leu Lys Glu Pro Leu Gln Gln Pro Ala Phe Pro Phe
            50                  55                  60

Ala Val Ala Asn Gln Leu Leu Leu Val Ser Leu Leu Glu His Leu Ser
65                  70                  75                  80

His Val His Glu Pro Asn Pro Leu Arg Ser Arg Gln Val Phe Lys Leu
                85                  90                  95

Leu Cys Gln Thr Phe Ile Lys Met Gly Leu Leu Ser Ser Phe Thr Cys
                100                 105                 110
```

```
Ser Asp Glu Phe Ser Ser Leu Arg Leu His His Asn Arg Ala Ile Thr
        115             120             125

His Leu Met Arg Ser Ala Arg Glu Arg Val Arg Gln Asp Pro Cys Ala
    130             135             140

Asp Asn Ser His Ile Gln Lys Ile Arg Ser Arg Glu Val Ala Leu Glu
145             150             155             160

Ala Gln Thr Ser Arg Tyr Leu Asn Glu Phe Glu Glu Leu Ser Ile Leu
            165             170             175

Gly Lys Gly Gly Tyr Gly Arg Val Tyr Lys Val Arg Asn Lys Leu Asp
            180             185             190

Gly Gln Tyr Tyr Ala Ile Lys Lys Ile Leu Ile Lys Gly Ala Thr Lys
        195             200             205

Thr Asp Cys Met Lys Val Leu Arg Glu Val Lys Val Leu Ala Gly Leu
    210             215             220

Gln His Pro Asn Ile Val Gly Tyr His Thr Ala Trp Ile Glu His Val
225             230             235             240

His Val His Val Gln Ala Asp Arg Val Pro Ile Gln Leu Pro Ser Leu
            245             250             255

Glu Val Leu Ser Asp Gln Glu Glu Asp Arg Asp Gln Tyr Gly Val Lys
            260             265             270

Asn Asp Ala Ser Ser Ser Ser Ser Ile Ile Phe Ala Glu Phe Ser Pro
        275             280             285

Glu Lys Glu Lys Ser Ser Asp Glu Cys Ala Val Glu Ser Gln Asn Asn
    290             295             300
```

```
Lys Leu Val Asn Tyr Thr Thr Asn Leu Val Val Arg Asp Thr Gly Glu
305               310               315               320

Phe Glu Ser Ser Thr Glu Arg Gln Glu Asn Gly Ser Ile Val Glu Arg
                325               330               335

Gln Leu Leu Phe Gly His Asn Ser Asp Val Glu Glu Asp Phe Thr Ser
            340               345               350

Ala Glu Glu Ser Ser Glu Glu Asp Leu Ser Ala Leu Arg His Thr Glu
        355               360               365

Val Gln Tyr His Leu Met Leu His Ile Gln Met Gln Leu Cys Glu Leu
    370               375               380

Ser Leu Trp Asp Trp Ile Ala Glu Arg Asn Arg Arg Ser Arg Glu Cys
385               390               395               400

Val Asp Glu Ser Ala Cys Pro Tyr Val Met Val Ser Val Ala Thr Lys
            405               410               415

Ile Phe Gln Glu Leu Val Glu Gly Val Phe Tyr Ile His Asn Met Gly
            420               425               430

Ile Val His Arg Asp Leu Lys Pro Arg Asn Ile Phe Leu His Gly Pro
        435               440               445

Asp Gln Gln Val Lys Ile Gly Asp Phe Gly Leu Ala Cys Ala Asp Ile
        450               455               460

Ile Gln Lys Asn Ala Ala Arg Thr Ser Arg Asn Gly Glu Arg Ala Pro
465               470               475               480

Thr His Thr Ser Arg Val Gly Thr Cys Leu Tyr Ala Ser Pro Glu Gln
                485               490               495
```

```
Leu Glu Gly Ser Glu Tyr Asp Ala Lys Ser Asp Met Tyr Ser Val Gly
            500                 505             510

Val Ile Leu Leu Glu Leu Phe Gln Pro Phe Gly Thr Glu Met Glu Arg
        515             520                 525

Ala Glu Val Leu Thr Gly Val Arg Ala Gly Arg Ile Pro Asp Ser Leu
    530                 535             540

Ser Lys Arg Cys Pro Ala Gln Ala Lys Tyr Val Gln Leu Leu Thr Arg
545                 550             555                     560

Arg Asn Ala Ser Gln Arg Pro Ser Ala Leu Gln Leu Leu Gln Ser Glu
            565             570                     575

Leu Phe Gln Asn Ser Ala His Val Asn Leu Thr Leu Gln Met Lys Ile
            580             585                 590

Ile Glu Gln Glu Arg Glu Ile Glu Glu Leu Lys Lys Gln Leu Ser Leu
        595             600                 605

Leu Ser Gln Ala Arg Gly Val Arg Ser Asp Arg Arg Asp Gly Glu Leu
    610                 615                 620

Pro Ala
625
```

## Claims

1. A pharmaceutical composition comprising heme-regulated eukaryotic initiation factor 2α kinase or the kinase modified in the heme binding region in the 170 N-terminal amino acids or the kinase insertion sequence to decrease regulation by heme in combination with a suitable pharmaceutical carrier for administration to a patient wherein the kinase or modified kinase is in a dosage effective to induce cellular differentiation, inhibit cellular proliferation or inhibit infection.

2. The pharmaceutical composition of claim 1 wherein the kinase before modification consists of (amino acids 1-626 of Sequence ID No. 2)

MetLeuGlyGlySerAlaGlyThrArgGlyGlyGluAlaGluGlyAspGlyAla
GlyAlaValGlyAlaValAlaProProProAlaIleAspPheProAlaGluVal
SerAspProLysTyrAspGluSerAspValProAlaGluLeuGlnValLeuLys
GluProLeuGlnGlnProAlaPheProPheAlaValAlaAsnGlnLeuLeuLeu
ValSerLeuLeuGluHisLeuSerHisValHisGluProAsnProLeuArgSer
ArgGlnValPheLysLeuLeuCysGlnThrPheIleLysMetGlyLeuLeuSer
SerPheThrCysSerAspGluPheSerSerLeuArgLeuHisHisAsnArgAla
IleThrHisLeuMetArgSerAlaArgGluArgValArgGlnAspProCysAla
AspAsnSerHisIleGlnLysIleArgSerArgGluValAlaLeuGluAlaGln
ThrSerArgTyrLeuAsnGluPheGluGluLeuSerIleLeuGlyLysGlyGly
TyrGlyArgValTyrLysValArgAsnLysLeuAspGlyGlnTyrTyrAlaIle
LysLysIleLeuIleLysGlyAlaThrLysThrAspCysMetLysValLeuArg
GluValLysValLeuAlaGlyLeuGlnHisProAsnIleValGlyTyrHisThr
AlaTrpIleGluHisValHisValHisValGlnAlaAspArgValProIleGln
LeuProSerLeuGluValLeuSerAspGlnGluGluAspArgAspGlnTyrGly
ValLysAsnAspAlaSerSerSerSerSerIleIlePheAlaGluPheSerPro
GluLysGluLysSerSerAspGluCysAlaValGluSerGlnAsnAsnLysLeu
ValAsnTyrThrThrAsnLeuValValArgAspThrGlyGluPheGluSerSer
ThrGluArgGlnGluAsnGlySerIleValGluArgGlnLeuLeuPheGlyHis
AsnSerAspValGluGluAspPheThrSerAlaGluGluSerSerGluGluAsp
LeuSerAlaLeuArgHisThrGluValGlnTyrHisLeuMetLeuHisIleGln
MetGlnLeuCysGluLeuSerLeuTrpAspTrpIleAlaGluArgAsnArgArg
SerArgGluCysValAspGluSerAlaCysProTyrValMetValSerValAla
ThrLysIlePheGlnGluLeuValGluGlyValPheTyrIleHisAsnMetGly
IleValHisArgAspLeuLysProArgAsnIlePheLeuHisGlyProAspGln

GlnValLysIleGlyAspPheGlyLeuAlaCysAlaAspIleIleGlnLysAsn
AlaAlaArgThrSerArgAsnGlyGluArgAlaProThrHisThrSerArgVal
GlyThrCysLeuTyrAlaSerProGluGlnLeuGluGlySerGluTyrAspAla
LysSerAspMetTyrSerValGlyValIleLeuLeuGluLeuPheGlnProPhe
GlyThrGluMetGluArgAlaGluValLeuThrGlyValArgAlaGlyArgIle
ProAspSerLeuSerLysArgCysProAlaGlnAlaLysTyrValGlnLeuLeu
ThrArgArgAsnAlaSerGlnArgProSerAlaLeuGlnLeuLeuGlnSerGlu
LeuPheGlnAsnSerAlaHisValAsnLeuThrLeuGlnMetLysIleIleGlu
GlnGluArgGluIleGluGluLeuLysLysGlnLeuSerLeuLeuSerGlnAla
ArgGlyValArgSerAspArgArgAspGlyGluLeuProAla

or any variant thereof which has a IF-2α kinase activity.

3. The composition of claim 1 wherein the first 170 amino acids of the kinase before modification consists of (amino acids 1-170 of Sequence ID No. 2)

```
MetLeuGlyGlySerAlaGlyThrArgGlyGlyGluAlaGluGlyAspGly
AlaGlyAlaValGlyAlaValAlaProProProAlaIleAspPheProAla
GluValSerAspProLysTyrAspGluSerAspValProAlaGluLeuGln
ValLeuLysGluProLeuGlnGlnProAlaPheProPheAlaValAlaAsn

GlnLeuLeuLeuValSerLeuLeuGluHisLeuSerHisValHisGluPro
AsnProLeuArgSerArgGlnValPheLysLeuLeuCysGlnThrPheIle
LysMetGlyLeuLeuSerSerPheThrCysSerAspGluPheSerSerLeu
ArgLeuHisHisAsnArgAlaIleThrHisLeuMetArgSerAlaArgGlu
ArgValArgGlnAspProCysAlaAspAsnSerHisIleGlnLysIleArg
SerArgGluValAlaLeuGluAlaGlnThrSerArgTyrLeuAsnGluPhe
```

or any variant thereof which has a IF-2α kinase activity.

4. A composition according to any one of claims 1 to 3 for use in medicine.

5. Use of a heme-regulated eukaryotic initiation factor 2α kinase or the kinase modified in the heme binding region in the 170 N-terminal amino acids or the kinase insertion sequence to decrease regulation by heme in the manufacture of a medicament for inducing cellular differentiation or for inhibiting cellular proliferation in a patient.

6. The use as in claim 5 wherein the heme-regulated eukaryotic initiation factor 2α kinase is expressed from a nucleic acid sequence having homology of greater than 80% and less than 100% to nucleotides 113 to 1990 of Sequence ID No. 1, in the following sequence:

ATGCTGGGGGGCAGCGCCGGGACCCGCGGGGGCGAAGCCGAGGGCGACGGGGCG
GGGGCGGTGGGGGCGGTGGCCCCGCCGCCCGCCATCGACTTCCCCGCTGAGGTG
TCGGATCCCAAGTATGACGAGTCGGATGTCCCGGCAGAGCTGCAGGTGCTGAAG
GAGCCGCTGCAGCAGCCAGCCTTCCCCTTCGCCGTCGCCAACCAGCTGCTGCTC
GTCTCCCTGCTGGAGCACCTGAGTCATGTGCACGAGCCAAACCCGCTTCGCTCC
AGACAGGTGTTTAAACTGCTCTGTCAGACCTTCATCAAAATGGGGCTGCTGTCT

TCCTTCACCTGCAGCGACGAGTTTAGCTCATTGAGGCTGCATCACAACAGAGCT
ATTACGCATCTGATGAGGTCCGCCAGAGAGAGAGTTCGGCAGGATCCCTGTGCT
GATAATTCTCATATCCAGAAAATCAGGTCGCGAGAAGTTGCCTTGGAAGCACAG
ACCTCACGATACTTGAATGAGTTTGAAGAGCTCTCCATCCTGGGGAAAGGTGGC
TATGGCCGAGTGTACAAGGTCAGGAATAAATTAGATGGCCAGTATTATGCAATT

AAAAAAATTCTGATTAAAGGTGCAACTAAAACAGATTGCATGAAGGTATTACGA
GAAGTGAAAGTGCTGGCGGGCCTCCAGCACCCTAATATCGTAGGCTATCACACC
GCGTGGATAGAGCATGTCCACGTTCACGTTCAAGCAGACAGAGTTCCGATTCAG
TTGCCTTCTCTGGAAGTGCTCTCTGACCAGGAAGAAGACAGAGATCAATATGGT
GTTAAAAATGATGCAAGCAGCAGCTCATCCATTATTTTCGCTGAGTTCTCCCCA
GAAAAAGAAAAATCCTCTGACGAATGTGCCGTTGAGAGTCAGAATAACAAACTG
GTGAACTACACCACCAACTTAGTGGTGAGGGACACCGGTGAGTTTGAATCGTCC
ACGGAGCGCCAAGAGAACGGCTCGATCGTGGAGCGTCAGCTACTGTTCGGGCAT
AACTCAGACGTAGAAGAGGATTTCACGTCCGCGGAGGAATCTTCTGAGGAAGAC
TTAAGCGCGTTGCGGCACACAGAGGTGCAGTACCACCTGATGCTGCATATCCAG
ATGCAGCTGTGCGAGCTGTCCCTGTGGGACTGGATCGCCGAGAGGAACAGGCGG
AGCCGAGAGTGCGTGGACGAATCTGCCTGTCCTTATGTTATGGTCAGTGTTGCA
ACAAAAATTTTTCAAGAACTGGTGGAAGGTGTGTTTTACATACATAACATGGGC
ATCGTGCACAGAGACCTGAAGCCTAGAAATATTTTTCTTCATGGTCCTGATCAA
CAAGTGAAAATAGGAGACTTTGGTCTGGCCTGCGCCGACATCATCCAGAAGAAT
GCGGCCCGGACCAGCAGAAACGGGGAGAGAGCACCCACACACTTCCCGAGTG
GGCACCTGTCTGTACGCCTCGCCCGAGCAGTTGGAAGGATCGGAGTATGATGCC
AAGTCAGACATGTACAGCGTCGGCGTGATCCTGCTGGAGCTCTTCCAGCCCTTC

GGGACAGAGATGGAGCGGGCAGAGGTCCTGACGGGCGTGCGAGCTGGCCGCATA
CCCGACTCCCTCAGTAAGAGGTGCCCGGCGCAGGCCAAGTACGTCCAGCTGCTG
ACCAGGAGGAACGCGTCCCAGCGGCCGTCCGCCCTTCAGCTGCTGCAGAGTGAG
CTCTTCCAGAACTCCGCGCATGTTAACCTCACCCTACAGATGAAGATAATAGAG
CAGGAAAGAGAAATCGAGGAACTCAAGAAGCAGCTGAGCCTCCTCTCCCAGGCC
CGAGGGGTGAGGAGTGACAGGCGAGACGGAGAGCTCCCTGCCTA.

7. Use of heme-regulated eukaryotic initiation factor 2α kinase or the kinase modified in the heme binding region in the 170 N-terminal amino acids or the kinase insertion sequence to decrease regulation by heme in the manufacture of a medicament for inhibiting infection in a patient.

8. Use of a heme-regulated eukaryotic initiation factor 2α kinase or the kinase modified in the heme binding region in the 170 N-terminal amino acids or the kinase insertion sequence to decrease regulation by heme in the manufacture of a medicament for regulating cell proliferation in a patient.

**Patentansprüche**

1. Pharmazeutische Zusammensetzung, die die Häm-regulierte Kinase des eukaryontischen Initiationsfaktors 2α oder die zur Erniedrigung der Häm-Regulation im Häm-bindenden Abschnitt innerhalb der 170 N-terminalen Aminosäuren oder in der Kinase-Insertionssequenz modifizierte Kinase aufweist, in Kombination mit einem geeigneten pharmazeutischen Träger für die Verabreichung an einen Patienten, wobei die Kinase oder die modifizierte Kinase in einer Dosis vorliegt, die wirksam ist, eine Zelldifferenzierung zu bewirken, eine Zellproliferation zu hemmen oder eine Infektion zu hemmen.

2. Pharmazeutische Zusammensetzung nach Anspruch 1, wobei die Kinase vor der Modifizierung besteht aus (Aminosäuren 1-626 der Sequenz ID Nr. 2)

```
MetLeuGlyGlySerAlaGlyThrArgGlyGlyGluAlaGluGlyAspGlyAla
GlyAlaValGlyAlaValAlaProProProAlaIleAspPheProAlaGluVal
SerAspProLysTyrAspGluSerAspValProAlaGluLeuGlnValLeuLys
GluProLeuGlnGlnProAlaPheProPheAlaValAlaAsnGlnLeuLeuLeu
ValSerLeuLeuGluHisLeuSerHisValHisGluProAsnProLeuArgSer
ArgGlnValPheLysLeuLeuCysGlnThrPheIleLysMetGlyLeuLeuSer
SerPheThrCysSerAspGluPheSerSerLeuArgLeuHisHisAsnArgAla
IleThrHisLeuMetArgSerAlaArgGluArgValArgGlnAspProCysAla
AspAsnSerHisIleGlnLysIleArgSerArgGluValAlaLeuGluAlaGln
ThrSerArgTyrLeuAsnGluPheGluGluLeuSerIleLeuGlyLysGlyGly
TyrGlyArgValTyrLysValArgAsnLysLeuAspGlyGlnTyrTyrAlaIle
LysLysIleLeuIleLysGlyAlaThrLysThrAspCysMetLysValLeuArg
GluValLysValLeuAlaGlyLeuGlnHisProAsnIleValGlyTyrHisThr
AlaTrpIleGluHisValHisValHisValGlnAlaAspArgValProIleGln
LeuProSerLeuGluValLeuSerAspGlnGluGluAspArgAspGlnTyrGly
ValLysAsnAspAlaSerSerSerSerSerIleIlePheAlaGluPheSerPro
GluLysGluLysSerSerAspGluCysAlaValGluSerGlnAsnAsnLysLeu
ValAsnTyrThrThrAsnLeuValValArgAspThrGlyGluPheGluSerSer
ThrGluArgGlnGluAsnGlySerIleValGluArgGlnLeuLeuPheGlyHis
```

```
AsnSerAspValGluGluAspPheThrSerAlaGluGluSerSerGluGluAsp
LeuSerAlaLeuArgHisThrGluValGlnTyrHisLeuMetLeuHisIleGln
MetGlnLeuCysGluLeuSerLeuTrpAspTrpIleAlaGluArgAsnArgArg
SerArgGluCysValAspGluSerAlaCysProTyrValMetValSerValAla
ThrLysIlePheGlnGluLeuValGluGlyValPheTyrIleHisAsnMetGly
IleValHisArgAspLeuLysProArgAsnIlePheLeuHisGlyProAspGln
GlnValLysIleGlyAspPheGlyLeuAlaCysAlaAspIleIleGlnLysAsn
AlaAlaArgThrSerArgAsnGlyGluArgAlaProThrHisThrSerArgVal
GlyThrCysLeuTyrAlaSerProGluGlnLeuGluGlySerGluTyrAspAla
LysSerAspMetTyrSerValGlyValIleLeuLeuGluLeuPheGlnProPhe
GlyThrGluMetGluArgAlaGluValLeuThrGlyValArgAlaGlyArgIle
ProAspSerLeuSerLysArgCysProAlaGlnAlaLysTyrValGlnLeuLeu
ThrArgArgAsnAlaSerGlnArgProSerAlaLeuGlnLeuLeuGlnSerGlu
LeuPheGlnAsnSerAlaHisValAsnLeuThrLeuGlnMetLysIleIleGlu
GlnGluArgGluIleGluGluLeuLysLysGlnLeuSerLeuLeuSerGlnAla
ArgGlyValArgSerAspArgArgAspGlyGluLeuProAla
```

oder aus einer beliebigen Variante davon, die IF-2α-Kinase-Aktivität besitzt.

3. Zusammensetzung nach Anspruch 1, wobei die ersten 170 Aminosäuren der Kinase vor der Modifizierung bestehen aus (Aminosäuren 1-170 der Sequenz ID Nr. 2)

```
MetLeuGlyGlySerAlaGlyThrArgGlyGlyGluAlaGluGlyAspGly
AlaGlyAlaValGlyAlaValAlaProProProAlaIleAspPheProAla
GluValSerAspProLysTyrAspGluSerAspValProAlaGluLeuGln
ValLeuLysGluProLeuGlnGlnProAlaPheProPheAlaValAlaAsn

GlnLeuLeuLeuValSerLeuLeuGluHisLeuSerHisValHisGluPro
AsnProLeuArgSerArgGlnValPheLysLeuLeuCysGlnThrPheIle
LysMetGlyLeuLeuSerSerPheThrCysSerAspGluPheSerSerLeu
ArgLeuHisHisAsnArgAlaIleThrHisLeuMetArgSerAlaArgGlu
ArgValArgGlnAspProCysAlaAspAsnSerHisIleGlnLysIleArg
SerArgGluValAlaLeuGluAlaGlnThrSerArgTyrLeuAsnGluPhe
```

oder aus einer beliebigen Variante davon, die IF-2α-Kinase-Aktivität besitzt.

4. Zusammensetzung nach einem beliebigen der Ansprüche 1 bis 3 für die Verwendung in der Medizin.

5. Verwendung einer Häm-regulierten Kinase des eukaryontischen Initiationsfaktors 2α oder einer zur Erniedrigung der Häm-Regulation im Häm-bindenden Abschnitt innerhalb der 170 N-terminalen Aminosäuren oder in der Kinase-Insertionssequenz modifizierten Kinase bei der Herstellung eines Medikaments zur Bewirkung einer Zelldifferenzierung oder zur Hemmung einer Zellproliferation bei einem Patienten.

**6.** Verwendung nach Anspruch 5, wobei die Häm-regulierte Kinase des eukaryontischen Initiationsfaktors 2α mittels einer Nukleinsäure-Sequenz exprimiert wird, die eine Homologie von mehr als 80% und weniger als 100% bezüglich der Nukleotide 113 bis 1990 der Sequenz ID Nr. 1 in der folgenden Sequenz aufweist:

```
ATGCTGGGGGGCAGCGCCGGGACCCGCGGGGGCGAAGCCGAGGGCGACGGGGCG
GGGGCGGTGGGGGCGGTGGCCCCGCCGCCCGCCATCGACTTCCCCGCTGAGGTG
TCGGATCCCAAGTATGACGAGTCGGATGTCCCGGCAGAGCTGCAGGTGCTGAAG
GAGCCGCTGCAGCAGCCAGCCTTCCCCTTCGCCGTCGCCAACCAGCTGCTGCTC
GTCTCCCTGCTGGAGCACCTGAGTCATGTGCACGAGCCAAACCCGCTTCGCTCC
AGACAGGTGTTTAAACTGCTCTGTCAGACCTTCATCAAAATGGGGCTGCTGTCT

TCCTTCACCTGCAGCGACGAGTTTAGCTCATTGAGGCTGCATCACAACAGAGCT
ATTACGCATCTGATGAGGTCCGCCAGAGAGAGAGTTCGGCAGGATCCCTGTGCT
GATAATTCTCATATCCAGAAAATCAGGTCGCGAGAAGTTGCCTTGGAAGCACAG
ACCTCACGATACTTGAATGAGTTTGAAGAGCTCTCCATCCTGGGGAAAGGTGGC
TATGGCCGAGTGTACAAGGTCAGGAATAAATTAGATGGCCAGTATTATGCAATT

AAAAAAATTCTGATTAAAGGTGCAACTAAAACAGATTGCATGAAGGTATTACGA
GAAGTGAAAGTGCTGGCGGGCCTCCAGCACCCTAATATCGTAGGCTATCACACC
GCGTGGATAGAGCATGTCCACGTTCACGTTCAAGCAGACAGAGTTCCGATTCAG
TTGCCTTCTCTGGAAGTGCTCTCTGACCAGGAAGAAGACAGAGATCAATATGGT
GTTAAAAATGATGCAAGCAGCAGCTCATCCATTATTTTCGCTGAGTTCTCCCCA
GAAAAAGAAAAATCCTCTGACGAATGTGCCGTTGAGAGTCAGAATAACAAACTG
GTGAACTACACCACCAACTTAGTGGTGAGGGACACCGGTGAGTTTGAATCGTCC
ACGGAGCGCCAAGAGAACGGCTCGATCGTGGAGCGTCAGCTACTGTTCGGGCAT
AACTCAGACGTAGAAGAGGATTTCACGTCCGCGGAGGAATCTTCTGAGGAAGAC
TTAAGCGCGTTGCGGCACACAGAGGTGCAGTACCACCTGATGCTGCATATCCAG
ATGCAGCTGTGCGAGCTGTCCCTGTGGGACTGGATCGCCGAGAGGAACAGGCGG
AGCCGAGAGTGCGTGGACGAATCTGCCTGTCCTTATGTTATGGTCAGTGTTGCA
ACAAAAATTTTTCAAGAACTGGTGGAAGGTGTGTTTTACATACATAACATGGGC
ATCGTGCACAGAGACCTGAAGCCTAGAAATATTTTTCTTCATGGTCCTGATCAA
CAAGTGAAAATAGGAGACTTTGGTCTGGCCTGCGCCGACATCATCCAGAAGAAT
GCGGCCCGGACCAGCAGAAACGGGGAGAGAGCACCCACACACTTCCCGAGTG
```

```
GGCACCTGTCTGTACGCCTCGCCCGAGCAGTTGGAAGGATCGGAGTATGATGCC
AAGTCAGACATGTACAGCGTCGGCGTGATCCTGCTGGAGCTCTTCCAGCCCTTC
GGGACAGAGATGGAGCGGGCAGAGGTCCTGACGGGCGTGCGAGCTGGCCGCATA
CCCGACTCCCTCAGTAAGAGGTGCCCGGCGCAGGCCAAGTACGTCCAGCTGCTG
ACCAGGAGGAACGCGTCCCAGCGGCCGTCCGCCCTTCAGCTGCTGCAGAGTGAG
CTCTTCCAGAACTCCGCGCATGTTAACCTCACCCTACAGATGAAGATAATAGAG
CAGGAAAGAGAAATCGAGGAACTCAAGAAGCAGCTGAGCCTCCTCTCCCAGGCC
CGAGGGGTGAGGAGTGACAGGCGAGACGGAGAGCTCCCTGCCTA.
```

7. Verwendung einer Häm-regulierten Kinase des eukaryontischen Initiationsfaktors 2α oder einer zur Erniedrigung der Häm-Regulation im Häm-bindenden Abschnitt innerhalb der 170 N-terminalen Aminosäuren oder in der Kinase-Insertionssequenz modifizierten Kinase bei der Herstellung eines Medikaments zur Hemmung einer Infektion bei einem Patienten.

8. Verwendung einer Häm-regulierten Kinase des eukaryontischen Initiationsfaktors 2α oder einer zur Erniedrigung der Häm-Regulation im Häm-bindenden Abschnitt innerhalb der 170 N-terminalen Aminosäuren oder in der Kinase-Insertionssequenz modifizierten Kinase bei der Herstellung eines Medikaments zur Regulierung der Zellproliferation bei einem Patienten.

**Revendications**

1. Composition pharmaceutique comprenant une kinase du facteur d'initiation 2α eucaryote à régulation par l'hème ou la kinase modifiée dans la région de fixation de l'hème dans les 170 acides aminés N-terminaux ou la séquence d'insertion de la kinase pour diminuer la régulation par l'hème en combinaison avec un véhicule pharmaceutique approprié pour une administration à un patient, dans laquelle la kinase ou la kinase modifiée est à une dose efficace pour induire une différenciation cellulaire, inhiber une prolifération cellulaire ou inhiber une infection.

2. Composition pharmaceutique selon la revendication 1, dans laquelle la kinase avant modification est constituée de (acides aminés 1 à 626 de la SEQ ID N° 2) :

```
MetLeuGlyGlySerAlaGlyThrArgGlyGlyGluAlaGluGlyAspGlyAla
GlyAlaValGlyAlaValAlaProProProAlaIleAspPheProAlaGluVal
SerAspProLysTyrAspGluSerAspValProAlaGluLeuGlnValLeuLys
GluProLeuGlnGlnProAlaPheProPheAlaValAlaAsnGlnLeuLeuLeu
ValSerLeuLeuGluHisLeuSerHisValHisGluProAsnProLeuArgSer
ArgGlnValPheLysLeuLeuCysGlnThrPheIleLysMetGlyLeuLeuSer
SerPheThrCysSerAspGluPheSerSerLeuArgLeuHisHisAsnArgAla
IleThrHisLeuMetArgSerAlaArgGluArgValArgGlnAspProCysAla
AspAsnSerHisIleGlnLysIleArgSerArgGluValAlaLeuGluAlaGln
ThrSerArgTyrLeuAsnGluPheGluGluLeuSerIleLeuGlyLysGlyGly
```

```
TyrGlyArgValTyrLysValArgAsnLysLeuAspGlyGlnTyrTyrAlaIle
LysLysIleLeuIleLysGlyAlaThrLysThrAspCysMetLysValLeuArg
GluValLysValLeuAlaGlyLeuGlnHisProAsnIleValGlyTyrHisThr
AlaTrpIleGluHisValHisValHisValGlnAlaAspArgValProIleGln
LeuProSerLeuGluValLeuSerAspGlnGluGluAspArgAspGlnTyrGly
ValLysAsnAspAlaSerSerSerSerIleIlePheAlaGluPheSerPro
GluLysGluLysSerSerAspGluCysAlaValGluSerGlnAsnAsnLysLeu
ValAsnTyrThrThrAsnLeuValValArgAspThrGlyGluPheGluSerSer
ThrGluArgGlnGluAsnGlySerIleValGluArgGlnLeuLeuPheGlyHis
AsnSerAspValGluGluAspPheThrSerAlaGluGluSerSerGluGluAsp
LeuSerAlaLeuArgHisThrGluValGlnTyrHisLeuMetLeuHisIleGln
MetGlnLeuCysGluLeuSerLeuTrpAspTrpIleAlaGluArgAsnArgArg
SerArgGluCysValAspGluSerAlaCysProTyrValMetValSerValAla
ThrLysIlePheGlnGluLeuValGluGlyValPheTyrIleHisAsnMetGly
IleValHisArgAspLeuLysProArgAsnIlePheLeuHisGlyProAspGln

GlnValLysIleGlyAspPheGlyLeuAlaCysAlaAspIleIleGlnLysAsn
AlaAlaArgThrSerArgAsnGlyGluArgAlaProThrHisThrSerArgVal
GlyThrCysLeuTyrAlaSerProGluGlnLeuGluGlySerGluTyrAspAla
LysSerAspMetTyrSerValGlyValIleLeuLeuGluLeuPheGlnProPhe
GlyThrGluMetGluArgAlaGluValLeuThrGlyValArgAlaGlyArgIle
ProAspSerLeuSerLysArgCysProAlaGlnAlaLysTyrValGlnLeuLeu
ThrArgArgAsnAlaSerGlnArgProSerAlaLeuGlnLeuLeuGlnSerGlu
LeuPheGlnAsnSerAlaHisValAsnLeuThrLeuGlnMetLysIleIleGlu
GlnGluArgGluIleGluGluLeuLysLysGlnLeuSerLeuLeuSerGlnAla
ArgGlyValArgSerAspArgArgAspGlyGluLeuProAla
```

ou d'une quelconque variante de cette séquence qui a une activité de kinase IF-2α.

3. Composition selon la revendication 1, dans laquelle les 170 premiers acides aminés de la kinase avant modification sont constitués de (acides aminés 1 à 170 de la SEQ ID N° 2) :

```
MetLeuGlyGlySerAlaGlyThrArgGlyGlyGluAlaGluGlyAspGly
AlaGlyAlaValGlyAlaValAlaProProProAlaIleAspPheProAla
GluValSerAspProLysTyrAspGluSerAspValProAlaGluLeuGln
ValLeuLysGluProLeuGlnGlnProAlaPheProPheAlaValAlaAsn

GlnLeuLeuLeuValSerLeuLeuGluHisLeuSerHisValHisGluPro
AsnProLeuArgSerArgGlnValPheLysLeuLeuCysGlnThrPheIle
LysMetGlyLeuLeuSerSerPheThrCysSerAspGluPheSerSerLeu
ArgLeuHisHisAsnArgAlaIleThrHisLeuMetArgSerAlaArgGlu
ArgValArgGlnAspProCysAlaAspAsnSerHisIleGlnLysIleArg
SerArgGluValAlaLeuGluAlaGlnThrSerArgTyrLeuAsnGluPhe
```

ou d'une quelconque variante de cette séquence qui a une activité de kinase IF-2α.

**4.** Composition selon l'une quelconque des revendications 1 à 3, pour une utilisation en médecine.

**5.** Utilisation d'une kinase du facteur d'initiation 2α eucaryote à régulation par l'hème ou de la kinase modifiée dans la région de fixation de l'hème dans les 170 acides aminés N-terminaux ou de la séquence d'insertion de la kinase pour diminuer la régulation par l'hème dans la fabrication d'un médicament pour induire une différenciation cellulaire ou pour inhiber une prolifération cellulaire chez un patient.

**6.** Utilisation selon la revendication 5, dans laquelle la kinase du facteur d'initiation 2α eucaryote à régulation par l'hème est exprimée à partir d'une séquence d'acides nucléiques ayant une homologie supérieure à 80 % et inférieure à 100 % avec les nucléotides 113 à 1990 de la SEQ ID N° 1, dans la séquence suivante :

```
ATGCTGGGGGGCAGCGCCGGGACCCGCGGGGGCGAAGCCGAGGGCGACGGGGCG
GGGGCGGTGGGGCGGTGGCCCCGCCGCCCGCCATCGACTTCCCCGCTGAGGTG
TCGGATCCCAAGTATGACGAGTCGGATGTCCCGGCAGAGCTGCAGGTGCTGAAG
GAGCCGCTGCAGCAGCCAGCCTTCCCCTTCGCCGTCGCCAACCAGCTGCTGCTC
GTCTCCCTGCTGGAGCACCTGAGTCATGTGCACGAGCCAAACCCGCTTCGCTCC
AGACAGGTGTTTAAACTGCTCTGTCAGACCTTCATCAAAATGGGGCTGCTGTCT

TCCTTCACCTGCAGCGACGAGTTTAGCTCATTGAGGCTGCATCACAACAGAGCT
ATTACGCATCTGATGAGGTCCGCCAGAGAGAGAGTTCGGCAGGATCCCTGTGCT
GATAATTCTCATATCCAGAAAATCAGGTCGCGAGAAGTTGCCTTGGAAGCACAG
ACCTCACGATACTTGAATGAGTTTGAAGAGCTCTCCATCCTGGGGAAAGGTGGC
TATGGCCGAGTGTACAAGGTCAGGAATAAATTAGATGGCCAGTATTATGCAATT

AAAAAAATTCTGATTAAAGGTGCAACTAAAACAGATTGCATGAAGGTATTACGA
GAAGTGAAAGTGCTGGCGGGCCTCCAGCACCCTAATATCGTAGGCTATCACACC
GCGTGGATAGAGCATGTCCACGTTCACGTTCAAGCAGACAGAGTTCCGATTCAG
TTGCCTTCTCTGGAAGTGCTCTCTGACCAGGAAGAAGACAGAGATCAATATGGT
GTTAAAAATGATGCAAGCAGCAGCTCATCCATTATTTTCGCTGAGTTCTCCCCA
GAAAAAGAAAAATCCTCTGACGAATGTGCCGTTGAGAGTCAGAATAACAAACTG
GTGAACTACACCACCAACTTAGTGGTGAGGGACACCGGTGAGTTTGAATCGTCC
ACGGAGCGCCAAGAGAACGGCTCGATCGTGGAGCGTCAGCTACTGTTCGGGCAT
AACTCAGACGTAGAAGAGGATTTCACGTCCGCGGAGGAATCTTCTGAGGAAGAC
TTAAGCGCGTTGCGGCACACAGAGGTGCAGTACCACCTGATGCTGCATATCCAG
ATGCAGCTGTGCGAGCTGTCCCTGTGGGACTGGATCGCCGAGAGGAACAGGCGG
AGCCGAGAGTGCGTGGACGAATCTGCCTGTCCTTATGTTATGGTCAGTGTTGCA
ACAAAAATTTTTCAAGAACTGGTGGAAGGTGTGTTTTACATACATAACATGGGC
ATCGTGCACAGAGACCTGAAGCCTAGAAATATTTTTCTTCATGGTCCTGATCAA
CAAGTGAAAATAGGAGACTTTGGTCTGGCCTGCGCCGACATCATCCAGAAGAAT
GCGGCCCGGACCAGCAGAAACGGGGAGAGAGCACCCACACACTTCCCGAGTG
GGCACCTGTCTGTACGCCTCGCCCGAGCAGTTGGAAGGATCGGAGTATGATGCC
AAGTCAGACATGTACAGCGTCGGCGTGATCCTGCTGGAGCTCTTCCAGCCCTTC
GGGACAGAGATGGAGCGGGCAGAGGTCCTGACGGGCGTGCGAGCTGGCCGCATA
CCCGACTCCCTCAGTAAGAGGTGCCCGGCGCAGGCCAAGTACGTCCAGCTGCTG
ACCAGGAGGAACGCGTCCCAGCGGCCGTCCGCCCTTCAGCTGCTGCAGAGTGAG


CTCTTCCAGAACTCCGCGCATGTTAACCTCACCCTACAGATGAAGATAATAGAG
CAGGAAAGAGAAATCGAGGAACTCAAGAAGCAGCTGAGCCTCCTCTCCCAGGCC
CGAGGGGTGAGGAGTGACAGGCGAGACGGAGAGCTCCCTGCCTA.
```

**7.** Utilisation de kinase du facteur d'initiation 2$\alpha$ eucaryote à régulation par l'hème ou de la kinase modifiée dans la région de fixation de l'hème dans les 170 acides aminés N-terminaux ou de la séquence d'insertion de la kinase pour diminuer la régulation par l'hème dans la fabrication d'un médicament pour inhiber une infection chez un patient.

**8.** Utilisation d'une kinase du facteur d'initiation 2$\alpha$ eucaryote à régulation par l'hème ou de la kinase modifiée dans la région de fixation de l'hème dans les 170 acides aminés N-terminaux ou de la séquence d'insertion de la kinase pour diminuer la régulation par l'hème dans la fabrication d'un médicament pour réguler la prolifération cellulaire chez un patient.

## FIG. 1
### (PRIOR ART)

FIG. 2

KINASE CONSERVED CATALYTIC DOMAINS : I ⟶ XI

```
                  I                            II                         III
CaMPK   9/TEEYQLFEEL   GKGAFSVVRR   CVKVLAGQEY   AAKIINTKKL   SARDHQKLER   EARICRLLKH
HRK     166/LNEFEELSIL GKGGYGRVYK   VRNKLDGQYY   AIKKILIKGA   TKTDCMKVLR   EVKVLAGLQH
Src     60/HEDVSLGELL  CKCNFGEVYK   GTLKDKTP.V   AVRTCKEDLP   .QELKIKFLQ   EAKILKQYDH


                IV                                   V
CaMPK   PNIVRLHDSI   SEEGHH....   ..YLIFDLVT   GGELFEDIVA   REY.YSEAD..   //..ASHCI
HRI     PNIVGYHTAW   IEHVHVHVQA   DRVPIQLPSL   EVLSDQEEDR   DQYGVKNDA    (138) ATKIF
Src     PNIVKLIGVC   TQRQPV....   ..YIIMELVP   GGDFLSFLRK   RKDELKLKQ.   //..LVRFS


                          VI                         VII
CaMPK   QQILEAVLHO   HQMGVVHRDL   KPENULLASK   LKGAAVKLAD   EGLAIEVEGE   QQAW......
HRI     QELVEGVFYI   HNMGIVHRDL   KPRNIFLHGP   DQQ..VKIGD   EGLACADIIQ   KNAARTSRNG
Src     LDVAAGMLYL   EGKNCIHRDL   AARNOLVGEN   NT...LKISD   FGMSRQEDGG   VYSSS.....


                VIII                            IX
CaMPK   ......FGFA   GTPGYLSPEV   LRKDPYGKPV   DLWNCGVILY   ILLVGYPPFW   DEDQHRLYQQ
HRI     ERAPTHTSRV   GTCLYASPEQ   LEGSEYDAKS   DMYSVGVILL   ELFQPFGTEM   ERAEVLTGVR
Src     ......GLKQ   IPIKWTAPEA   LNYGRYSSES   DVWSFGILLW   ETFSLGVCPY   PGMTNQQARE


                X                               XI
CaMPK   IKAGAYDFPS   PEWDT......   VTPEAKDLI   NKMLTINPSK   RITAAEALKH   /210
HRI     AGRIPDSLSK   RCPAQ (26)    FQNSAHVNU   TLQMKIIEQE   REIEELKKQL   /20
src     QVERGYRMSA   PQN........   CPEEIFTIM   MKCWDYKPEN   RPKFSDLHKE   /9
```

FIGURE 3

FIG. 4a

FIG. 4b

# FIG. 5A

Mouse

# FIG. 5B

Human

# FIG. 6